# EUROPEAN PATENT APPLICATION

(11) **EP 4 052 725 A1**
(43) Date of publication of application: **07.09.2022**
(21) Application number: 20882559.6
(22) Date of filing: 20.10.2020
(51) Int. Cl.: A61K 39/395, A61P 11/00, A61P 29/00, A61P 43/00, C07K 16/18

(54) **PROPHYLACTIC AND/OR THERAPEUTIC AGENT FOR INFLAMMATORY PULMONARY DISEASE**

(30) Priority: 30.10.2019 JP 2019197222
(71) Applicant: National University Corporation Okayama University, Kita-ku, Okayama-shi, Okayama 700-8530 (JP)
(72) Inventor: SAKAGUCHI, Masakiyo, Okayama-shi, Okayama 700-8530 (JP); TOYOOKA, Shinichi, Okayama-shi, Okayama 700-8530 (JP); KINOSHITA, Rie, Okayama-shi, Okayama 700-8530 (JP); ARAKI, Kota, Okayama-shi, Okayama 700-8530 (JP)
(74) Representative: Arnold & Siedsma
(86) International application number: PCT/JP2020/039460
(87) International publication number: WO 2021/085252

(57) **Abstract**

The present invention provides an inflammatory pulmonary disease agent capable of effectively preventing and/or treating an inflammatory pulmonary disease, and specifically relates to a prophylactic and/or therapeutic agent for an inflammatory pulmonary disease containing, as an active ingredient, an antibody or an antibody fragment having antigen-binding activity for an S100A8/A9 heterodimer. The inflammatory pulmonary disease can be effectively prevented and/or treated by blocking interaction between S100A8/A9 and a group of receptors therefor. Specifically, the inflammatory pulmonary disease can be effectively prevented and/or treated by: blocking interaction between S100A8/A9 and RAGE, which is a receptor therefor, to suppress: the expression of NF-κB, which is a transcription factor present downstream of RAGE and induces the expression of various inflammatory cytokines; the proliferation of activated fibroblasts; and the differentiation of activated fibroblasts into myofibroblasts. In addition, the prophylactic and/or therapeutic agent for an inflammatory pulmonary disease of the present invention may also be suitably used as a prophylactic and/or therapeutic agent for COVID-19.

SEQUENCE LISTING

<110>  National University Corporation Okayama University

<120>  Prophylactic and/or therapeutic agent for inflammatory pulmonary
       disease

<130>  GP20-1005PCT

<150>  JP P2019-197222
<151>  2019-10-30

<160>  39

<170>  PatentIn version 3.5

<210>  1
<211>  20
<212>  DNA
<213>  Artificial

<220>
<223>  Fwd primer for TNF alpha

<400>  1
gacaagcctg tagcccatgt                                                   20


<210>  2
<211>  18
<212>  DNA
<213>  Artificial

<220>
<223>  Rev primer for TNF alpha

<400>  2
tctcagctcc acgccatt                                                     18


<210>  3
<211>  18
<212>  DNA
<213>  Artificial

<220>
<223>  Fwd primer for IL-6

<400>  3
cttccctgcc ccagtacc                                                     18


<210>  4
<211>  21
<212>  DNA
<213>  Artificial

<220>
<223>  Rev primer for IL-6

<400>  4
ctgaagaggt gagtggctgt c                                                 21

<210>  5
<211>  20
<212>  DNA
<213>  Artificial

<220>
<223>  Fwd primer for IL-8

<400>  5
agacagcaga gcacacaagc                                                    20


<210>  6
<211>  19
<212>  DNA
<213>  Artificial

<220>
<223>  Rev primer for IL-8

<400>  6
aggaaggctg ccaagagag                                                     19


<210>  7
<211>  5
<212>  PRT
<213>  Artificial

<220>
<223>  No.45:CDR H1

<400>  7

Ser Tyr Trp Met Gln
1               5


<210>  8
<211>  17
<212>  PRT
<213>  Artificial

<220>
<223>  No.45:CDR H2

<400>  8

Ala Ile Tyr Pro Gly Asp Gly Asp Thr Arg Asp Thr Gln Lys Phe Lys
1               5                   10                  15


Gly



<210>  9
<211>  10
<212>  PRT
<213>  Artificial

<220>

<223>  No.45:CDR H3

<400>  9

Met Ala Gly Tyr Asn Tyr Asp Asn Asp Tyr
1               5                   10


<210>  10
<211>  6
<212>  PRT
<213>  Artificial

<220>
<223>  No.85:CDR H1

<400>  10

Ser Gly Tyr Asn Trp His
1               5


<210>  11
<211>  16
<212>  PRT
<213>  Artificial

<220>
<223>  No.85:CDR H2

<400>  11

Tyr Ile Gln Tyr Ser Gly Ser Thr Asn Tyr Asn Pro Ser Leu Lys Ser
1               5                   10                  15


<210>  12
<211>  11
<212>  PRT
<213>  Artificial

<220>
<223>  No.85:CDR H3

<400>  12

Ala Leu Arg Tyr Asp Tyr Ser Trp Phe Ala Tyr
1               5                   10


<210>  13
<211>  5
<212>  PRT
<213>  Artificial

<220>
<223>  No.235:CDR H1

<400>  13

Asn Phe Trp Met Asn
1               5

<210>  14
<211>  17
<212>  PRT
<213>  Artificial

<220>
<223>  No.235:CDR H2

<400>  14

Gln Ile Tyr Pro Gly Lys Ser Asp Thr Asn Tyr Asn Gly Lys Phe Lys
1               5                   10                  15

Gly

<210>  15
<211>  14
<212>  PRT
<213>  Artificial

<220>
<223>  No.235:CDR H3

<400>  15

Trp Gly Ala Tyr Tyr Lys Tyr Gly Gly Ser Tyr Phe Asp Tyr
1               5                   10

<210>  16
<211>  7
<212>  PRT
<213>  Artificial

<220>
<223>  No.258:CDR H1

<400>  16

Thr Ala Ser Met Gly Val Ser
1               5

<210>  17
<211>  16
<212>  PRT
<213>  Artificial

<220>
<223>  No.258:CDR H2

<400>  17

His Ile Tyr Trp Asp Asp Asp Lys Arg Tyr Asn Pro Ser Leu Lys Ser
1               5                   10                  15

<210>  18
<211>  8

<212>  PRT
<213>  Artificial

<220>
<223>  No.258:CDR H3

<400>  18

Arg Pro Leu Gly Tyr Phe Asp Val
1               5


<210>  19
<211>  5
<212>  PRT
<213>  Artificial

<220>
<223>  No.260:CDR H1

<400>  19

Asn Tyr Gly Val His
1               5


<210>  20
<211>  16
<212>  PRT
<213>  Artificial

<220>
<223>  No.260:CDR H2

<400>  20

Val Val Trp Ala Gly Gly Ser Thr Asn Tyr Asn Ser Ala Leu Met Ser
1               5                   10                  15


<210>  21
<211>  13
<212>  PRT
<213>  Artificial

<220>
<223>  No.260:CDR H3

<400>  21

Ala Arg Asp Tyr Tyr Gly Tyr Asp Gly Tyr Phe Gly Ala
1               5                   10


<210>  22
<211>  11
<212>  PRT
<213>  Artificial

<220>
<223>  No.45: CDR L1

<400>  22

Lys Ala Ser Gln Asp Ile Asn Lys Tyr Ile Ala
1               5                   10

<210>  23
<211>  7
<212>  PRT
<213>  Artificial

<220>
<223>  No.45: CDR L2

<400>  23

Tyr Thr Ser Thr Leu Gln Pro
1               5

<210>  24
<211>  8
<212>  PRT
<213>  Artificial

<220>
<223>  No.45: CDR L3

<400>  24

Leu Gln Tyr Asp Asn Leu Arg Thr
1               5

<210>  25
<211>  11
<212>  PRT
<213>  Artificial

<220>
<223>  No.85: CDR L1

<400>  25

Lys Ala Ser Gln Asp Val Ser Thr Ala Val Ala
1               5                   10

<210>  26
<211>  7
<212>  PRT
<213>  Artificial

<220>
<223>  No.85: CDR L2

<400>  26

Ser Ala Ser Tyr Arg Tyr Thr
1               5

<210>  27
<211>  9

<212>  PRT
<213>  Artificial

<220>
<223>  No.85: CDR L3

<400>  27

Gln Gln His Tyr Ser Thr Pro Leu Thr
1               5


<210>  28
<211>  11
<212>  PRT
<213>  Artificial

<220>
<223>  No.235: CDR L1

<400>  28

Ser Ala Ser Gln Gly Ile Ser Asn Tyr Leu Asn
1               5                   10


<210>  29
<211>  7
<212>  PRT
<213>  Artificial

<220>
<223>  No.235: CDR L2

<400>  29

Tyr Thr Ser Ser Leu His Ser
1               5


<210>  30
<211>  9
<212>  PRT
<213>  Artificial

<220>
<223>  No.235: CDR L3

<400>  30

Gln Gln Tyr Ser Lys Phe Pro Tyr Thr
1               5


<210>  31
<211>  11
<212>  PRT
<213>  Artificial

<220>
<223>  No.258: CDR L1

<400>  31

Lys Ala Ser Gln Asp Ile Asn Asn Tyr Ile Ser
1               5                   10


<210>  32
<211>  7
<212>  PRT
<213>  Artificial

<220>
<223>  No.258: CDR L2

<400>  32

Tyr Thr Ser Thr Leu Gln Pro
1               5


<210>  33
<211>  9
<212>  PRT
<213>  Artificial

<220>
<223>  No.258: CDR L3

<400>  33

Leu Gln Tyr Asp Asn Leu Leu Trp Thr
1               5


<210>  34
<211>  11
<212>  PRT
<213>  Artificial

<220>
<223>  No.260: CDR L1

<400>  34

Lys Ala Ser Gln Asp Ile Asn Ser Tyr Leu Thr
1               5                   10


<210>  35
<211>  7
<212>  PRT
<213>  Artificial

<220>
<223>  No.260: CDR L2

<400>  35

Arg Ala Asn Arg Leu Val Asp
1               5


<210>  36
<211>  9

<212>  PRT
<213>  Artificial

<220>
<223>  No.260: CDR L3

<400>  36

Leu Gln Tyr Asp Glu Phe Pro Leu Thr
1               5


<210>  37
<211>  22
<212>  DNA
<213>  Artificial Sequence

<220>
<223>  NF-kB consensus binding motif

<400>  37
agttgagggg actttcccag gc                                                22


<210>  38
<211>  20
<212>  DNA
<213>  Artificial Sequence

<220>
<223>  Fwd primer for TMPRSS2

<400>  38
gatgacagcg gatccaccag                                                   20


<210>  39
<211>  20
<212>  DNA
<213>  Artificial Sequence

<220>
<223>  Rev primer for TMPRSS2

<400>  39
ccgcaggcta tacagcgtaa                                                   20

## Description

The present invention relates to a prophylactic and/or therapeutic agent for an inflammatory pulmonary disease containing, as an active ingredient, an antibody or an antibody fragment having antigen-binding activity for a heterodimer of an S100A8 protein (sometimes referred to simply as "S100A8") and an S100A9 protein (sometimes referred to simply as "S100A9").

The present application claims priority from Japanese Patent Application No. 2019-197222, which is incorporated herein by reference.

### Background Art

S100 proteins are each a calcium-binding protein that is expressed in a cell-type-specific manner and has two EF-hands, and 20 kinds of subfamilies thereof have been recognized heretofore. S100A8 (MRP8, calgranulin A) is a member of the calcium-binding protein S100 family, and is usually coexpressed with S100A9 (MRP14, calgranulin B). S100A8/A9 (calprotectin), which is a heterodimer of S100A8 and S100A9, is considered to accumulate in body fluid during inflammation, thereby being involved in the onset of a human chronic inflammatory disease, such as rheumatoid arthritis (RA), cystic fibrosis, Crohn's disease, ulcerative colitis, allergic dermatitis, or an infection.

S100A8/A9 is, for example, secreted by the lungs, and has a function of attracting distant cancer cells and a function of forming, in the lungs, an immune-suppressive environment appropriate for settlement and proliferation of cancer cells. As the group of receptors for S100A8/A9, there are known, for example, EMMPRIN, neuroplastin-α (NPTNα), NPTNβ, M-cell adhesion molecule (MCAM), and activated leukocyte cell adhesion molecule (ALCAM). There is a report of a screening method for a chronic inflammation suppressor or a cancer metastasis suppressor based on binding inhibition with a focus on EMMPRIN among the receptors for S100A8/A9 (Patent Literature 1). In Patent Literature 1, it is shown that EMMPRIN is a receptor particularly for

S100A9, and there is a disclosure that results of screening have found Japanese mugwort extract, dong quai extract, white dead-nettle extract, and the like to inhibit binding between EMMPRIN and S100A9. There is a report of a screening method for a cell proliferation suppressor based on binding inhibition with a focus on NPTN among the receptors for S100A8/A9 (Patent Literature 2). In Patent Literature 2, there is a disclosure that results of screening have found Japanese mugwort extract, glycyrrhiza extract, ginseng extract, and the like to inhibit binding between NPTN and S100A8. Compounds regarded as S100-inhibitors have been reported to be useful for treatment of, for example, cancer, autoimmune diseases, inflammatory diseases and neurodegenerative diseases (Patent Literature 3). In addition, there is also a report of usefulness of S100A9 as a biomarker for inflammatory bowel disease (Patent Literature 4).

Further, receptor for advanced glycation end products (RAGE) is also known as a receptor for S100A8/A9 (Non Patent Literature 1). In Non Patent Literature 1, there is a report that S100A8/A9 binds to TLR4 and RAGE on the membrane of BV-2 microglial cells, and stimulates NF-κB in the BV-2 microglial cells via ERV and JNK mediation to enhance its activity (Non Patent Literature 1).

The number of patients suffering from inflammatory diseases in the lungs, that is, diseases such as idiopathic interstitial pneumonias (IIPs) typified by idiopathic pulmonary fibrosis (IPF) (hereinafter referred to as "inflammatory pulmonary diseases") continues to increase. Of the idiopathic interstitial pneumonias, idiopathic pulmonary fibrosis is a disease found all over the world, is not responsive to steroids or immunosuppressive agents, and has an extremely poor prognosis with an average survival period of 2 months or less after acute exacerbation. As therapeutic drugs for idiopathic pulmonary fibrosis, there are known two kinds of molecularly targeted therapeutic drugs (pirfenidone and nintedanib) (Non Patent Literatures 2 and 3). The reality is, however, that each of those drugs is symptomatic therapy, cannot be expected to suppress or ameliorate fibrosis, and cannot be said to be radical treatment.

### Citation List

### Patent Literature

[PTL 1] JP 2011-47932 A
[PTL 2] JP 2014-59210 A
[PTL 3] WO 2015/177367 A1
[PTL 4] JP 2016-217956 A

### Non Patent Literature

[NPL 1] Li Ma et al., INTERNATIONAL JOURNAL OF MOLECULAR MEDICINE (2017) 40: 31-38, DOI: 10.3892/ijmm.2017.2987
[NPL 2] Margaritopoulos et al., BMC Pulmonary Medicine (2018) 18:177; doi.org/10.1186/s12890-018-0736-z
[NPL 3] Lutz Wollin1 et al., European Respiratory Journal (2015); DOI: 10.1183/09031936.00174914

### Summary of Invention

### Technical Problem

An object of the present invention is to provide a medicament capable of effectively preventing and/or treating an inflammatory pulmonary disease. More specifically, the object is to provide a prophylactic and/or therapeutic agent for an inflammatory pulmonary disease containing, as an active ingredient, an antibody or an antibody fragment having antigen-binding activity for S100A8 and/or S100A9, which are known as inflammation-related proteins.

### Solution to Problem

In order to achieve the above-mentioned object, the inventors of the present invention have made extensive investigations with a focus on S100A8 and/or S100A9 and a group of receptors therefor (EMMPRIN, NPTNβ, MCAM, ALCAM, and RAGE), and as a result, have found that an inflammatory pulmonary disease can be effectively prevented and/or treated by blocking interaction between S100A8 and/or S100A9 and a group of receptors therefor. Thus, the inventors have completed the present invention.

That is, the present invention includes the following.
1. A prophylactic and/or therapeutic agent for an inflammatory pulmonary disease, including an antibody or an antibody fragment as an active ingredient, the antibody or the antibody fragment having antigen-binding activity for a heterodimer of an S100A8 protein and an S100A9 protein.
2. The prophylactic and/or therapeutic agent for an inflammatory pulmonary disease according to the above-mentioned item 1, wherein the antibody or the antibody fragment has antigen-binding activity for the heterodimer higher than antigen-binding activity for an S100A8 monomer.
3. The prophylactic and/or therapeutic agent for an inflammatory pulmonary disease according to the above-mentioned item 1 or 2, wherein the antibody or the antibody fragment has antigen-binding activity for the heterodimer of S100A8 and S1 00A9, and is free of antigen-binding activity for an S100A8 monomer and/or an S100A9 monomer.
4. The prophylactic and/or therapeutic agent for an inflammatory pulmonary disease according to any one of the above-mentioned items 1 to 3, wherein the antigen-binding activity is a neutralizing affinity.
5. The prophylactic and/or therapeutic agent for an inflammatory pulmonary disease according to any one of the above-mentioned items 1 to 4, wherein the antibody or the antibody fragment is a monoclonal antibody or a monoclonal antibody fragment.
6. The prophylactic and/or therapeutic agent for an inflammatory pulmonary disease according to the above-mentioned item 5, wherein a subclass of the monoclonal antibody is selected from the group consisting of IgG₁, IgG₂, IgG₃, and IgG₄.
7. The prophylactic and/or therapeutic agent for an inflammatory pulmonary disease according to any one of the above-mentioned items 1 to 6,
   wherein the antibody or the antibody fragment as the active ingredient is an antibody or an antibody fragment containing:
   heavy chain variable regions including a heavy chain variable region 1 (CDR HI), a heavy chain variable region 2 (CDR H2), and a heavy chain variable region 3 (CDR H3); and
   light chain variable regions including a light chain variable region 1 (CDR L1), a light chain variable region 2 (CDR L2), and a light chain variable region 3 (CDR L3),
   wherein the heavy chain variable region 1 (CDR H1) contains any one of the amino acid sequence set forth in SEQ ID NO: 7, SEQ ID NO: 10, SEQ ID NO: 13, SEQ ID NO: 16, or SEQ ID NO: 19, or the amino acid sequence having one or a plurality of amino acids deleted, added, substituted, or inserted in SEQ ID NO: 7, 10, 13, 16, or 19,
   wherein the heavy chain variable region 2 (CDR H2) contains any one of the amino acid sequence set forth in SEQ ID NO: 8, SEQ ID NO: 11, SEQ ID NO: 14, SEQ ID NO: 17, or SEQ ID NO: 20, or the amino acid sequence having one or a plurality of amino acids deleted, added, substituted, or inserted in SEQ ID NO: 8, 11, 14, 17, or 20,
   wherein the heavy chain variable region 3 (CDR H3) contains any one of the amino acid sequence set forth in SEQ ID NO: 9, SEQ ID NO: 12, SEQ ID NO: 15, SEQ ID NO: 18, or SEQ ID NO: 21, or the amino acid sequence having one or a plurality of amino acids deleted, added, substituted, or inserted in SEQ ID NO: 9, 12, 15, 18, or 21,
   wherein the light chain variable region 1 (CDR L1) contains any one of the amino acid sequence set forth in SEQ ID NO: 22, SEQ ID NO: 25, SEQ ID NO: 28, SEQ ID NO: 31, or SEQ ID NO: 34, or the amino acid sequence having one or a plurality of amino acids deleted, added, substituted, or inserted in SEQ ID NO: 22, 25, 28, 31, or 34,
   wherein the light chain variable region 2 (CDR L2) contains any one of the amino acid sequence set forth in SEQ ID NO: 23, SEQ ID NO: 26, SEQ ID NO: 29, SEQ ID NO: 32, or SEQ ID NO: 35, or the amino acid sequence having one or a plurality of amino acids deleted, added, substituted, or inserted in SEQ ID NO: 23, 26, 29, 32, or 35, and
   wherein the light chain variable region 3 (CDR L3) contains any one of the amino acid sequence set forth in SEQ ID NO: 24, SEQ ID NO: 27, SEQ ID NO: 30, SEQ ID NO: 33, or SEQ ID NO: 36, or the amino acid sequence having one or a plurality of amino acids deleted, added, substituted, or inserted in SEQ ID NO: 24, 27, 30, 33, or 36.
8. The prophylactic and/or therapeutic agent for an inflammatory pulmonary disease according to the above-mentioned item 7,
   wherein the heavy chain variable region 1 (CDR H1) contains any one of the amino acid sequence set forth in SEQ ID NO: 7, or the amino acid sequence having one or a plurality of amino acids deleted, added, substituted, or inserted in the amino acid sequence set forth in SEQ ID NO: 7,
   wherein the heavy chain variable region 2 (CDR H2) contains any one of the amino acid sequence set forth in SEQ ID NO: 8, or the amino acid sequence having one or a plurality of amino acids deleted, added, substituted, or inserted in the amino acid sequence set forth in SEQ ID NO: 8,
   wherein the heavy chain variable region 3 (CDR H3) contains any one of the amino acid sequence set forth in SEQ ID NO: 9, or the amino acid sequence having one or a plurality of amino acids deleted, added, substituted, or inserted in the amino acid sequence set forth in SEQ ID NO: 9,
   wherein the light chain variable region 1 (CDR L1) contains any one of the amino acid sequence set forth in SEQ ID NO: 22, or the amino acid sequence having one or a plurality of amino acids deleted, added, substituted, or inserted in the amino acid sequence set forth in SEQ ID NO: 22,
   wherein the light chain variable region 2 (CDR L2) contains any one of the amino acid sequence set forth in SEQ ID NO: 23, or the amino acid sequence having one or a plurality of amino acids deleted, added, substituted, or inserted in the amino acid sequence set forth in SEQ ID NO: 23, and
   wherein the light chain variable region 3 (CDR L3) contains any one of the amino acid sequence set forth in SEQ ID NO: 24, or the amino acid sequence having one or a plurality of amino acids deleted, added, substituted, or inserted in the amino acid sequence set forth in SEQ ID NO: 24.
9. The prophylactic and/or therapeutic agent for an inflammatory pulmonary disease according to the above-mentioned item 7,
   wherein the heavy chain variable region 1 (CDR H1) contains any one of the amino acid sequence set forth in SEQ ID NO: 10, or the amino acid sequence having one or a plurality of amino acids deleted, added, substituted, or inserted in the amino acid sequence set forth in SEQ ID NO: 10,
   wherein the heavy chain variable region 2 (CDR H2) contains any one of the amino acid sequence set forth in SEQ ID NO: 11, or the amino acid sequence having one or a plurality of amino acids deleted, added, substituted, or inserted in the amino acid sequence set forth in SEQ ID NO: 11,
   wherein the heavy chain variable region 3 (CDR H3) contains any one of the amino acid sequence set forth in SEQ ID NO: 12, or the amino acid sequence having one or a plurality of amino acids deleted, added, substituted, or inserted in the amino acid sequence set forth in SEQ ID NO: 12,
   wherein the light chain variable region 1 (CDR L1) contains any one of the amino acid sequence set forth in SEQ ID NO: 25, or the amino acid sequence having one or a plurality of amino acids deleted, added, substituted, or inserted in the amino acid sequence set forth in SEQ ID NO: 25,
   wherein the light chain variable region 2 (CDR L2) contains any one of the amino acid sequence set forth in SEQ ID NO: 26, or the amino acid sequence having one or a plurality of amino acids deleted, added, substituted, or inserted in the amino acid sequence set forth in SEQ ID NO: 26, and
   wherein the light chain variable region 3 (CDR L3) contains any one of the amino acid sequence set forth in SEQ ID NO: 27, or the amino acid sequence having one or a plurality of amino acids deleted, added, substituted, or inserted in the amino acid sequence set forth in SEQ ID NO: 27.
10. The prophylactic and/or therapeutic agent for an inflammatory pulmonary disease according to the above-mentioned item 7,
   wherein the heavy chain variable region 1 (CDR H1) contains any one of the amino acid sequence set forth in SEQ ID NO: 13, or the amino acid sequence having one or a plurality of amino acids deleted, added, substituted, or inserted in the amino acid sequence set forth in SEQ ID NO: 13,
   wherein the heavy chain variable region 2 (CDR H2) contains any one of the amino acid sequence set forth in SEQ ID NO: 14, or the amino acid sequence having one or a plurality of amino acids deleted, added, substituted, or inserted in the amino acid sequence set forth in SEQ ID NO: 14,
   wherein the heavy chain variable region 3 (CDR H3) contains any one of the amino acid sequence set forth in SEQ ID NO: 15, or the amino acid sequence having one or a plurality of amino acids deleted, added, substituted, or inserted in the amino acid sequence set forth in SEQ ID NO: 15,
   wherein the light chain variable region 1 (CDR L1) contains any one of the amino acid sequence set forth in SEQ ID NO: 28, or the amino acid sequence having one or a plurality of amino acids deleted, added, substituted, or inserted in the amino acid sequence set forth in SEQ ID NO: 28,
   wherein the light chain variable region 2 (CDR L2) contains any one of the amino acid sequence set forth in SEQ ID NO: 29, or the amino acid sequence having one or a plurality of amino acids deleted, added, substituted, or inserted in the amino acid sequence set forth in SEQ ID NO: 29, and
   wherein the light chain variable region 3 (CDR L3) contains any one of the amino acid sequence set forth in SEQ ID NO: 30, or the amino acid sequence having one or a plurality of amino acids deleted, added, substituted, or inserted in the amino acid sequence set forth in SEQ ID NO: 30.
11. The prophylactic and/or therapeutic agent for an inflammatory pulmonary disease according to the above-mentioned item 7,
   wherein the heavy chain variable region 1 (CDR H1) contains any one of the amino acid sequence set forth in SEQ ID NO: 16, or the amino acid sequence having one or a plurality of amino acids deleted, added, substituted, or inserted in the amino acid sequence set forth in SEQ ID NO: 16,
   wherein the heavy chain variable region 2 (CDR H2) contains any one of the amino acid sequence set forth in SEQ ID NO: 17, or the amino acid sequence having one or a plurality of amino acids deleted, added, substituted, or inserted in the amino acid sequence set forth in SEQ ID NO: 17,
   wherein the heavy chain variable region 3 (CDR H3) contains any one of the amino acid sequence set forth in SEQ ID NO: 18, or the amino acid sequence having one or a plurality of amino acids deleted, added, substituted, or inserted in the amino acid sequence set forth in SEQ ID NO: 18,
   wherein the light chain variable region 1 (CDR L1) contains any one of the amino acid sequence set forth in SEQ ID NO: 31, or the amino acid sequence having one or a plurality of amino acids deleted, added, substituted, or inserted in the amino acid sequence set forth in SEQ ID NO: 31,
   wherein the light chain variable region 2 (CDR L2) contains any one of the amino acid sequence set forth in SEQ ID NO: 32, or the amino acid sequence having one or a plurality of amino acids deleted, added, substituted, or inserted in the amino acid sequence set forth in SEQ ID NO: 32, and
   wherein the light chain variable region 3 (CDR L3) contains any one of the amino acid sequence set forth in SEQ ID NO: 33, or the amino acid sequence having one or a plurality of amino acids deleted, added, substituted, or inserted in the amino acid sequence set forth in SEQ ID NO: 33.
12. The prophylactic and/or therapeutic agent for an inflammatory pulmonary disease according to the above-mentioned item 7,
   wherein the heavy chain variable region 1 (CDR H1) contains any one of the amino acid sequence set forth in SEQ ID NO: 19, or the amino acid sequence having one or a plurality of amino acids deleted, added, substituted, or inserted in the amino acid sequence set forth in SEQ ID NO: 19,
   wherein the heavy chain variable region 2 (CDR H2) contains any one of the amino acid sequence set forth in SEQ ID NO: 20, or the amino acid sequence having one or a plurality of amino acids deleted, added, substituted, or inserted in the amino acid sequence set forth in SEQ ID NO: 20,
   wherein the heavy chain variable region 3 (CDR H3) contains any one of the amino acid sequence set forth in SEQ ID NO: 21, or the amino acid sequence having one or a plurality of amino acids deleted, added, substituted, or inserted in the amino acid sequence set forth in SEQ ID NO: 21,
   wherein the light chain variable region 1 (CDR L1) contains any one of the amino acid sequence set forth in SEQ ID NO: 34, or the amino acid sequence having one or a plurality of amino acids deleted, added, substituted, or inserted in the amino acid sequence set forth in SEQ ID NO: 34,
   wherein the light chain variable region 2 (CDR L2) contains any one of the amino acid sequence set forth in SEQ ID NO: 35, or the amino acid sequence having one or a plurality of amino acids deleted, added, substituted, or inserted in the amino acid sequence set forth in SEQ ID NO: 35, and
   wherein the light chain variable region 3 (CDR L3) contains any one of the amino acid sequence set forth in SEQ ID NO: 36, or the amino acid sequence having one or a plurality of amino acids deleted, added, substituted, or inserted in the amino acid sequence set forth in SEQ ID NO: 36.
13. The prophylactic and/or therapeutic agent for an inflammatory pulmonary disease according to any one of the above-mentioned items 1 to 12, wherein the prophylactic and/or therapeutic agent blocks interaction between S100A8 and/or S100A9 and RAGE, which is a receptor therefor, to suppress: expression of NF-κB, which is a transcription factor present downstream of RAGE and induces expression of inflammatory cytokines, and proliferation of lung fibroblasts; proliferation of activated fibroblasts; and differentiation of activated fibroblasts into myofibroblasts.
14. The prophylactic and/or therapeutic agent for an inflammatory pulmonary disease according to any one of the above-mentioned items 1 to 13, wherein the prophylactic and/or therapeutic agent serves as a prophylactic and/or therapeutic agent for COVID-19.

### Advantageous Effects of Invention

The prophylactic and/or therapeutic agent for an inflammatory pulmonary disease of the present invention can effectively prevent and/or treat the inflammatory pulmonary disease by blocking interaction between S100A8 and/or S100A9 and RAGE, which is one kind of receptor therefor. Here, RAGE is a major receptor for S100A8/A9 in the lungs, is highly expressed in alveolar epithelial cells, which cause the inflammatory pulmonary disease, and serves as the origin of a pathological condition associated with the inflammatory pulmonary disease. That is, the prophylactic and/or therapeutic agent for an inflammatory pulmonary disease of the present invention blocks interaction between S100A8 and/or S100A9 and RAGE, which is one kind of receptor therefor, to suppress: the expression of NF-κB, which is a transcription factor present downstream of RAGE and induces the expression of various inflammatory cytokines; the proliferation of activated fibroblasts; and the differentiation of activated fibroblasts into myofibroblasts. On the basis of the action mechanism described above, the prophylactic and/or therapeutic agent for an inflammatory pulmonary disease of the present invention can effectively prevent and/or treat the inflammatory pulmonary disease.

### Brief Description of Drawings

FIG. 1 is a diagram for illustrating the structure of an expression vector for preparing an S100A8/A9 heterodimer serving as an antigen for generating an anti-S 100A8/A9 antibody of the present invention (Reference Example 1).
FIG. 2 is a photograph showing results obtained by subjecting a purified S100A8/A9 heterodimer, S100A8 monomer, and S100A9 monomer to SDS-PAGE, followed by CBB staining (Reference Example 1).
FIG. 3 is a chart showing the results of HPLC analysis of the purified S100A8/A9 heterodimer, S100A8 monomer, and S100A9 monomer (Reference Example 1).
FIG. 4 is a diagram for illustrating the thermodynamic stabilities of the purified S100A8/A9 heterodimer, S100A8 monomer, and S100A9 monomer (Reference Example 1).
FIG. 5 is a graph showing the results of an investigation, by an ELISA method, of the neutralizing abilities of 10 clones selected from hybridomas for generating anti-S100A8/A9 antibodies against the S100A8/A9 heterodimer, S100A8, or S100A9 (Example 1).
FIG. 6 includes graphs showing the results of an investigation of the expression-suppressing actions of 10 clones selected from hybridomas for generating anti-S100A8/A9 antibodies on each of TNF-α, IL-6, and IL-8 through use of human keratinocytes having inflammatory cytokines strongly induced by S100A8/A9 (Example 2).
FIG. 7 is a diagram for illustrating the configuration of a chimeric antibody obtained by fusing the Fc portion of human IgG₂ to the Fab domain of an anti-S100A8/A9 monoclonal antibody (Clone No. 45) (Example 4).
FIG. 8 is a graph showing the proliferation enhancement of mouse fibroblasts by S100A8/A9 (Example 5).
FIG. 9 is a graph showing the proliferation enhancement of human fibroblasts by S100A8/A9 (Example 5).
FIG. 10 is a photograph showing the results of evaluation of S100A8/A9-dependent NF-κB signal activation in fibroblasts (Example 6).
FIG. 11 is a photograph showing the results of evaluation of NF-κB signal suppression by the anti-S100A8/A9 antibody (α-S100A8/A9 antibody) in fibroblasts (Example 6).
FIG. 12 includes photographs showing the results of evaluation of the expression induction of α-SMA by S100A8/A9 and the S100A8/A9-induced α-SMA expression-suppressing ability of the anti-S 1 00A8/A9 antibody (α-S100A8/A9 antibody) in human lung fibroblasts (Example 7).
FIG. 13 includes photographs showing the results of evaluation of the expression induction of α-SMA and collagen by S100A8/A9 and the S100A8/A9-induced α-SMA and collagen expression-suppressing abilities of the anti-S 100A8/A9 antibody (α-S100A8/A9 antibody) in mouse fibroblasts (Example 7).
FIG. 14 is a diagram for illustrating an experimental protocol for investigating the lung injury-suppressing effect of the anti-S 1 00A8/A9 antibody through use of a pulmonary fibrosis model intratracheally injected with bleomycin (Example 8).
FIG. 15 includes graphs showing the concentration-dependent body weight reduction-suppressing effect of anti-S100A8/A9 antibody injection in the pulmonary fibrosis model intratracheally injected with bleomycin (Example 8).
FIG. 16 includes photographs showing the results of lung CT scanning after anti-S100A8/A9 antibody injection in the pulmonary fibrosis model intratracheally injected with bleomycin (Example 8).
FIG. 17 is a figure showing a graph quantifying the area of the high density area of lung after anti-S100A8/A9 antibody injection in the pulmonary fibrosis model intratracheally injected with bleomycin (Example 8).
FIG. 18 is a graph showing the survival rates of mice after anti-S 1 00A8/A9 antibody injection in the pulmonary fibrosis model intratracheally injected with bleomycin (Example 8).
FIG. 19 is a graph showing the suppressing effect of the anti-S100A8/A9 antibody on TMPRSS2 expression in human lung cells (Example 9).

### Description of Embodiments

The present invention relates to a prophylactic and/or therapeutic agent for an inflammatory pulmonary disease containing, as an active ingredient, an antibody or an antibody fragment having antigen-binding activity for S100A8/A9. The "antibody having antigen-binding activity for S100A8/A9" contained in the prophylactic and/or therapeutic agent for an inflammatory pulmonary disease of the present invention is sometimes referred to as "anti-S100A8/A9 antibody" or alternatively as "antibody of the present invention". Herein, "S100A8/A9" means a complex of S100A8 and S100A9, which may sometimes be referred to as "S100A8/A9 heterodimer".

Herein, the antibody of the present invention is an antibody generated using the S100A8/A9 heterodimer as an antigen, and has antigen-binding activity for the S100A8/A9 heterodimer. The anti-S100A8/A9 antibody preferably has antigen-binding activity for the S100A8/A9 heterodimer higher than antigen-binding activity for an S100A8 monomer. Of the anti-S 1 00A8/A9 antibodies, an antibody having antigen-binding activity for the heterodimer of S100A8 and S100A9, and being free of antigen-binding activity for the S100A8 monomer and/or an S100A9 monomer is more suitable. The antigen-binding activity in the foregoing description only needs to be antigen-binding activity as generally understood, and is not particularly limited, but an example thereof may be a neutralizing antibody affinity. Further, the anti-S100A8/A9 antibody is suitably an antibody having a neutralizing antibody affinity for the heterodimer of S100A8 and S100A9 higher than a neutralizing antibody affinity for the S100A8 monomer, more suitably an antibody having a neutralizing antibody affinity for the heterodimer of S100A8 and S100A9, and being free of a neutralizing antibody affinity for the S100A8 monomer and/or the S100A9 monomer.

Herein, the term "antibody of the present invention" is used in its broadest sense, and encompasses monoclonal antibodies, polyclonal antibodies, chimeric antibodies, and multispecific antibodies as long as those antibodies each show the antigen-binding activity described above. The antibody of the present invention may contain a heavy chain variable region (VH-CDR) and/or a light chain variable region (VL-CDR), or a fragment thereof. The class of the antibody of the present invention refers to the type of constant domain or constant region included in a heavy chain (H chain) of the antibody, and examples thereof include IgA, IgD, IgE, IgG, and IgM. Herein, the class of the antibody is not particularly limited, but is most suitably IgG. As subclasses of IgG, there are given, for example, IgG₁, IgG₂, IgG₃, and IgG₄, among which IgG₁ or IgG₂ is suitable. Herein, the "antibody fragment" of the antibody having antigen-binding activity for the S100A8/A9 heterodimer only needs to be a fragment of an antibody having part of an antibody structure and retaining the activity of the antibody of the present invention described above. As the structure of the antibody fragment, there is given a fragment of the antigen-binding site of an antibody, and examples thereof include a heavy chain variable region (VH-CDR) and/or a light chain variable region (VL-CDR), and fragments thereof. Examples thereof may include Fv, Fab, Fab', Fab'-SH, F(ab')₂, and combinations thereof.

The antibody of the present invention may be a human antibody, a humanized antibody, or a chimeric antibody. The human antibody refers to: an antibody produced by a human or human cells; or an antibody including an amino acid sequence corresponding to the amino acid sequence of an antibody derived from a nonhuman supply source using a human antibody repertoire or other human antibody-coding sequences.

The amino acid sequences of VH-CDR and/or VL-CDR contained in the antibody of the present invention may contain, for example, amino acid sequences identified by the following SEQ ID NOs. For example, a heavy chain variable region 1 (CDR H1) may contain any one amino acid sequence set forth in SEQ ID NO: 7, SEQ ID NO: 10, SEQ ID NO: 13, SEQ ID NO: 16, or SEQ ID NO: 19. A heavy chain variable region 2 (CDR H2) may contain any one amino acid sequence set forth in SEQ ID NO: 8, SEQ ID NO: 11, SEQ ID NO: 14, SEQ ID NO: 17, or SEQ ID NO: 20. A heavy chain variable region 3 (CDR H3) may contain any one amino acid sequence set forth in SEQ ID NO: 9, SEQ ID NO: 12, SEQ ID NO: 15, SEQ ID NO: 18, or SEQ ID NO: 21. For example, a light chain variable region 1 (CDR L1) may contain any one amino acid sequence set forth in SEQ ID NO: 22, SEQ ID NO: 25, SEQ ID NO: 28, SEQ ID NO: 31, or SEQ ID NO: 34. A light chain variable region 2 (CDR L2) region may contain any one amino acid sequence set forth in SEQ ID NO: 23, SEQ ID NO: 26, SEQ ID NO: 29, SEQ ID NO: 32, or SEQ ID NO: 35. A light chain variable region 3 (CDR L3) may contain any one amino acid sequence set forth in SEQ ID NO: 24, SEQ ID NO: 27, SEQ ID NO: 30, SEQ ID NO: 33, or SEQ ID NO: 36. In the present invention, amino acid sequence information on each of the above-mentioned regions is also encompassed in the scope of rights. In addition to the above-mentioned amino acid sequences, even when one or a plurality of amino acids are substituted, deleted, added, or inserted in each of the sequences, anti-S100A8/A9 antibodies or antibody fragments containing such amino acid sequences are also encompassed in the scope of rights of the present invention as long as those antibodies or antibody fragments each show antigen-binding activity for the S100A8/A9 heterodimer.

The antibody or the antibody fragment of the present invention may be generated in accordance with a conventional method using the above-mentioned S100A8/A9 heterodimer antigen.

When the antibody of the present invention is a monoclonal antibody, hybridomas that produce the anti-S 100A8/A9 antibody may be obtained by immunizing a mammal, such as a mouse or a rat, with the S100A8/A9 heterodimer antigen, collecting lymphocytes from the animal, and fusing myeloma cells thereto in accordance with a conventional method to generate hybridomas. The mammal may be immunized in accordance with a conventional method. For example, the animal may be immunized using, as an immunogen, a mixture of the S100A8/A9 heterodimer antigen and an adjuvant. The adjuvant is not particularly limited, but examples thereof include Freund's complete adjuvant and Freund's incomplete adjuvant. A method of administering the immunogen at the time of the immunization may be any of the methods known *per se,* such as subcutaneous injection, intraperitoneal injection, intravenous injection, and intramuscular injection. Of those, subcutaneous injection or intraperitoneal injection is preferred. The immunization may be performed once or a plurality of times at an appropriate interval, preferably a plurality of times at an interval of from 1 week to 5 weeks. Cells that produce the monoclonal antibody of interest may be obtained by investigating a binding property to the S100A8/A9 heterodimer by an ELISA method or the like for a culture supernatant of the hybridomas generated above, and repeating operation of cloning antibody-producing hybridomas. A method known *per se* or the like may be applied as a method of generating a humanized antibody.

From the antibody-producing hybridoma cells, purification of total RNA and subsequent synthesis of cDNA may be performed in accordance with conventional methods. Through amplification of antibody genes for a full-length heavy chain (H chain) and light chain (L chain) from the resultant cDNA by PCR using respective primers, respective gene fragments may be obtained. Through ligation of the resultant gene fragments to an expression vector, the antibody genes may be cloned. With regard to the amino acid sequences of the H chain and L chain of the antibody, the base sequence of a plasmid vector encoding the amino acid sequences may be identified to determine the amino acid sequence of the antibody. On the basis of the obtained information on the amino acid sequence and the base sequence, the antibody may be generated by a gene recombination technique, or the antibody may be generated by a synthesis method. When the antibody is generated by a gene recombination technique, the antibody may be generated by, for example, a method described in WO 2017/061354 A1.

When the antibody is generated by a gene recombination technique, for example, information on genes encoding respective amino acids that identify CDR H1, CDR H2, CDR H3, CDR L1, CDR L2, and CDR L3 may be utilized. As a specific amino acid sequence, for example, for CDR H1, there is given any one amino acid sequence set forth in SEQ ID NO: 7, SEQ ID NO: 10, SEQ ID NO: 13, SEQ ID NO: 16, or SEQ ID NO: 19. For CDR H2, there is given any one amino acid sequence set forth in SEQ ID NO: 8, SEQ ID NO: 11, SEQ ID NO: 14, SEQ ID NO: 17, or SEQ ID NO: 20. For CDR H3, there is given any one amino acid sequence set forth in SEQ ID NO: 9, SEQ ID NO: 12, SEQ ID NO: 15, SEQ ID NO: 18, or SEQ ID NO: 21. For example, for CDR L1, there is given any one amino acid sequence set forth in SEQ ID NO: 22, SEQ ID NO: 25, SEQ ID NO: 28, SEQ ID NO: 31, or SEQ ID NO: 34. For CDR L2, there is given any one amino acid sequence set forth in SEQ ID NO: 23, SEQ ID NO: 26, SEQ ID NO: 29, SEQ ID NO: 32, or SEQ ID NO: 35. For CDR L3, there is given any one amino acid sequence set forth in SEQ ID NO: 24, SEQ ID NO: 27, SEQ ID NO: 30, SEQ ID NO: 33, or SEQ ID NO: 36. The present invention also encompasses base sequence information encoding respective amino acids that identify the above-identified CDR H1, CDR H2, CDR H3, CDR L1, CDR L2, and CDR L3 and base sequence information on strands complementary thereto. In the present invention, in addition to the above-mentioned base sequence information, even when a base sequence has one to a plurality of nucleotides substituted, deleted, added, or inserted, such base sequence information is also encompassed in the scope of rights of the present invention as long as the base sequence allows the anti-S100A8/A9 antibody of the present invention to be generated.

A screening method for the antibody of the present invention and investigation methods for evaluating the antibody are specifically described in, for example, Reference Example, Examples, and experimental examples to be described later, but for example, the following methods may also be applied.

Among the above-mentioned antibody-producing hybridomas, hybridomas expressing a plurality of kinds of S100A8/A9 neutralizing antibody candidates may be adapted to serum-free culture and prepared in large amounts for an *in vitro* or *in vivo* experiment. A culture supernatant of each clone may be recovered and subjected to the purification of the antibody. Methods known *per se* or any method to be developed in the future may be applied to the purification of the antibody. For example, the antibody may be recovered by performing affinity chromatography.

Specifically, affinity purification using Protein A/G is generally employed, and a column suitable for each animal species or antibody subclass may be used. A purity test for the purified antibody may be performed by a method known *per se,* and may be performed, for example, by CBB staining.

For evaluation of the antibody or the antibody fragment of the present invention, S100A8/A9-binding decoy protein formulations (exEMMPRIN-Fc, exNPTNβ-Fc, exMCAM-Fc, exRAGE-Fc, and exALCAM-Fc) serving as receptors for S100A8/A9 may be appropriately prepared.

Herein, the "inflammatory pulmonary disease" means any of pulmonary diseases in general that are inflammatory, such as idiopathic/chronic inflammatory pulmonary disease, bronchial asthma, and interstitial pneumonia, which can be prevented and/or treated by the prophylactic and/or therapeutic agent for an inflammatory pulmonary disease of the present invention, and examples thereof may include idiopathic interstitial pneumonias typified by idiopathic pulmonary fibrosis (IPF) (IIPs: including cryptogenic organizing pneumonia and nonspecific interstitial pneumonia (NSIP)), idiopathic nonspecific interstitial pneumonia, chronic obstructive pulmonary disease (COPD), hypersensitivity pneumonitis, and the novel coronavirus infectious disease (COVID-19). Of the above-mentioned inflammatory pulmonary diseases, for example, COPD and bronchial asthma have rapidly increased in recent years. According to recent statistical data, it is estimated that 3% to 6% of the total population of Japan suffer from bronchial asthma, and 8.5% suffer from COPD. Thus, the inflammatory pulmonary diseases significantly affect the healthy life expectancy of humans, and inflict immeasurable losses on social economy and health care financing. In the pathological condition of such inflammatory pulmonary disease, the differentiation of activated fibroblasts into myofibroblasts and the accompanying excessive expression, enhancement, and deposition of collagen and extracellular matrix components conceivably lead to the exacerbation of pulmonary fibrosis. In this connection, the prophylactic and/or therapeutic agent for an inflammatory pulmonary disease of the present invention can effectively prevent and/or treat the inflammatory pulmonary disease on the basis of an action mechanism to be described later.

The prophylactic and/or therapeutic agent for an inflammatory pulmonary disease of the present invention may be locally administered, or may be systemically administered. The antibody to be used in the prophylactic and/or therapeutic agent for an inflammatory pulmonary disease of the present invention may be used in combination with an antibody other than the antibody of the present invention having a pharmaceutically acceptable purity optionally with a pharmaceutically acceptable carrier, excipient, or stabilizer. In addition, the prophylactic and/or therapeutic agent for an inflammatory pulmonary disease of the present invention may be optionally prepared in a freeze-dried formulation or water-soluble form for storage. When prepared in a form for parenteral administration, the prophylactic and/or therapeutic agent for an inflammatory pulmonary disease of the present invention may include pharmaceutically acceptable, sterilized, aqueous or nonaqueous solutions, diluents, suspensions, and emulsions. Examples of the nonaqueous diluents are propylene glycol, polyethylene glycol, plant oils, such as olive oil, and organic ester compositions, such as ethyl oleate, which are suitable for injection. Aqueous carriers may include water, alcoholic/aqueous solutions, emulsions, suspensions, saline, and buffered media.

Parenteral carriers may include sodium chloride solution, Ringer's dextrose, dextrose, and sodium chloride, lactated Ringer's, and fixed oils. Intravenous carriers may include, for example, fluid replenishers, and nutrient and electrolyte replenishers (such as those based on Ringer's dextrose). The prophylactic and/or therapeutic agent for an inflammatory pulmonary disease of the present invention may further contain a preservative and other additives, such as an antimicrobial compound, an antioxidant, a chelating agent, and an inert gas. The above-mentioned components other than the antibody of the present invention serving as the active ingredient in the prophylactic and/or therapeutic agent for an inflammatory pulmonary disease of the present invention may be used alone or in appropriate combination thereof.

The prophylactic and/or therapeutic agent for an inflammatory pulmonary disease of the present invention may be used in combination with, as required, one kind or two or more kinds of physiologically active compounds, preferably one kind or two or more kinds of an anti-inflammatory agent known *per se,* an anti-inflammatory agent to be developed in the future, and for example, any other medicaments, capable of alleviating a side effect that have complementary activities that do not adversely affect each other.

The prophylactic and/or therapeutic agent for an inflammatory pulmonary disease of the present invention contains a therapeutically effective amount of the anti-S100A8/A9 antibody. The "therapeutically effective amount" refers to an amount effective, at dosages and for periods of time necessary, to achieve a desired therapeutic result. The therapeutically effective amount may be set in consideration of, for example, the disease state, age, sex, and body weight of an individual, and the efficacy of the pharmaceutical to elicit a desired response in the individual.

The prophylactic and/or therapeutic agent for an inflammatory pulmonary disease of the present invention may be used in the following manner: a single dose or divided doses thereof are used generally every 24 hours, 12 hours, 8 hours, 6 hours, 4 hours, or 2 hours or any combination thereof, generally at least once on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 after the start of treatment, or at least once in week 1, 2, 3, 4, 5, 6, 7, 8, 9, 10, 11, 12, 13, 14, 15, 16, 17, 18, 19, or 20, or any combination thereof, at a daily dose in terms of daily antibody amount of from about 0.1 mg/kg body weight to about 100 mg/kg body weight, for example, 0.5 mg/kg body weight, 0.9 mg/kg body weight, 1.0 mg/kg body weight, 1.1 mg/kg body weight, 1.5 mg/kg body weight, 2 mg/kg body weight, 3 mg/kg body weight, 4 mg/kg body weight, 5 mg/kg body weight, 6 mg/kg body weight, 7 mg/kg body weight, 8 mg/kg body weight, 9 mg/kg body weight, 10 mg/kg body weight, 11 mg/kg body weight, 12 mg/kg body weight, 13 mg/kg body weight, 14 mg/kg body weight, 15 mg/kg body weight, 16 mg/kg body weight, 17 mg/kg body weight, 18 mg/kg body weight, 19 mg/kg body weight, 20 mg/kg body weight, 21 mg/kg body weight, 22 mg/kg body weight, 23 mg/kg body weight, 24 mg/kg body weight, 25 mg/kg body weight, 26 mg/kg body weight, 27 mg/kg body weight, 28 mg/kg body weight, 29 mg/kg body weight, 30 mg/kg body weight, 40 mg/kg body weight, 45 mg/kg body weight, 50 mg/kg body weight, 60 mg/kg body weight, 70 mg/kg body weight, 80 mg/kg body weight, 90 mg/kg body weight, or 100 mg/kg body weight.

### Examples

Now, the results of experiments performed to complete the present invention are shown as Reference Example, and the present invention is more specifically described in Examples.

However, the present invention is not limited thereto, and various applications are possible without departing from the technical concept of the present invention.

### (Reference Example 1) Preparation of S100A8/A9 Heterodimer for Generating Anti-S100A8/A9 Antibodies

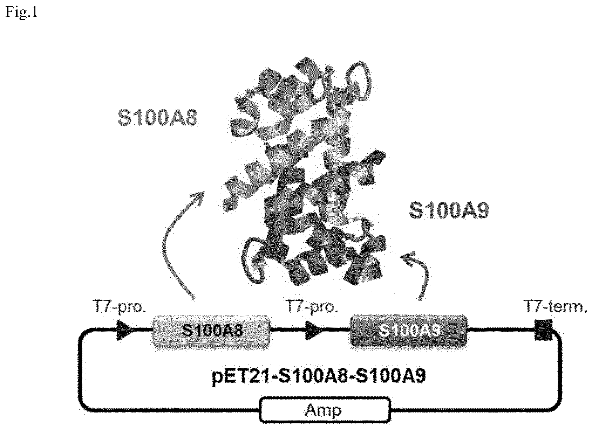

In this Reference Example, the preparation of an S100A8/A9 heterodimer serving as an antigen for the generation of anti-S100A8/A9 antibodies shown in subsequent Examples is described. The S100A8/A9 heterodimer was generated with *Escherichia coli* using an expression vector obtained by incorporating full-length S100A8 and full-length S100A9 into pET21 (see FIG. 1), and was purified (see Futami J. et al., Biochem Biophys Rep., 19; 6: 94-100 (2016)). For comparative examples, full-length S100A8 or full-length S100A9 was incorporated into pET21, generated with *Escherichia coli* by the same technique as above, and purified (see Futami J. et al. (2016)).

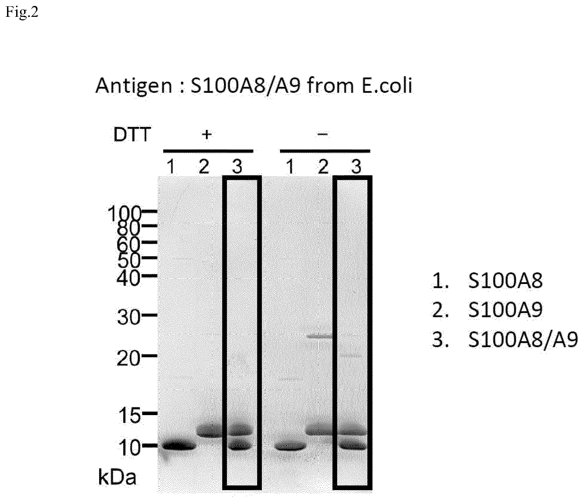

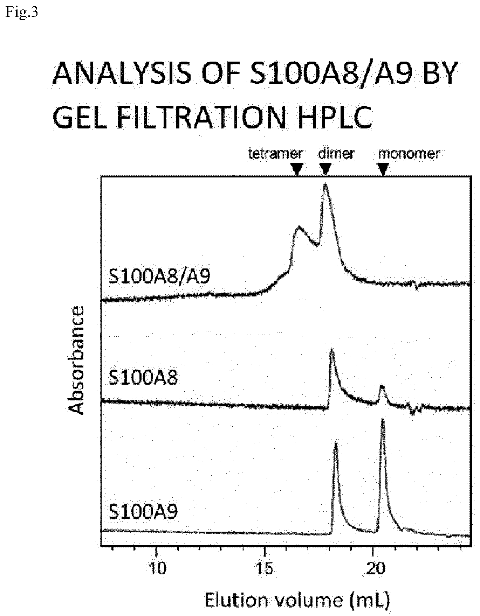

The purified S100A8/A9 heterodimer, and S100A8 monomer and S100A9 monomer serving as comparative examples were subjected to SDS-PAGE, followed by CBB staining. The results are shown in FIG. 2. The S100A8/A9 heterodimer had nearly equal amounts of S100A8 and S100A9, and hence was recognized to have been purified to a high purity. Further, the S100A8/A9 heterodimer was subjected to HPLC analysis. As a result, the results of comparison among the structures of S100A8, S100A9, and S100A8/A9 were as follows: only S100A8/A9 had no monomer presence recognized and mostly had a dimer structure (see FIG. 3). Meanwhile, S100A8 and S100A9 generated as comparative examples were each a mixture of a monomer and a dimer (see FIG. 3).

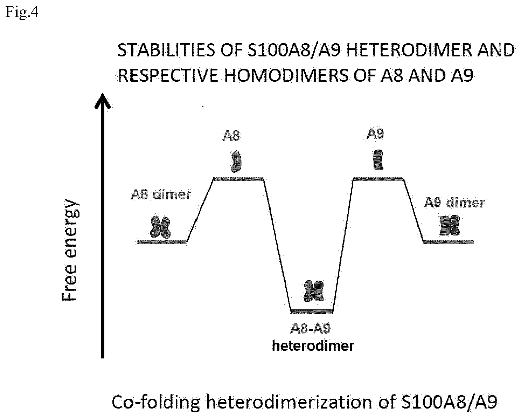

In FIG. 4, it is illustrated that a naturally occurring S100A8/A9 heterodimer (abbreviated simply as "A8-A9 heterodimer") is thermodynamically stable, but S100A8 (abbreviated simply as "A8") and S100A9 (abbreviated simply as "A9") each form a homodimer, and hence it is difficult to generate a stable S1 00A8/A9 heterodimer by mixing S100A8 and S100A9. The S1 00A8/A9 heterodimer prepared by the method of this Reference Example has high stability, and can be used as an S100A8/A9 heterodimer antigen for generating antibodies in Examples to be described later.

### (Example 1) Generation of Anti-S100A8/A9 Monoclonal Antibodies

In this Example, the generation of anti-S100A8/A9 monoclonal antibodies to be used in the following Examples and experimental examples is described. The anti-S100A8/A9 monoclonal antibodies of this Example were generated using the S100A8/A9 heterodimer prepared in the foregoing (Reference Example 1) as an antigen.

### (1) Generation of Hybridomas

The anti-S 1 00A8/A9 monoclonal antibodies of this Example were generated through use of the S100A8/A9 heterodimer prepared in the foregoing (Reference Example 1) as an antigen and through utilization of a monoclonal antibody on-contract service, GenoStaff (Nippon Genetics). Mice (BALB/c) were used as immunized animals, and Titer-MAX was used as an adjuvant in immunization with the antigen. In accordance with a conventional method, the spleen of the immunized animals and mouse myeloma cells (P3U1) were fused using polyethylene glycol (PEG1500) to generate hybridomas, affording 160 clones.

### (2) Cloning of Hybridomas and Generation of Antibodies

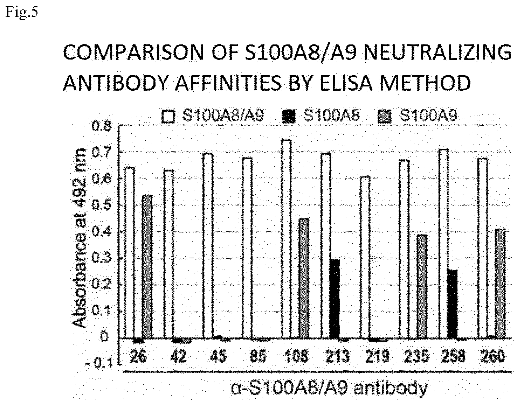

The 160 clones of hybridomas obtained above were subjected to ELISA screening by immobilizing the S100A8/A9 heterodimer, S100A8, or S100A9. Thus, 10 clones shown in FIG. 5 were selected. Hybridomas expressing the selected S100A8/A9 neutralizing antibody ("α-S100A8/A9 antibody" shown in FIG. 5) candidates were adapted to serum-free culture and prepared in large amounts for *in vitro* and *in vivo* experiments. A culture supernatant of each clone was recovered and purified with a Protein G column to prepare several milligrams of protein for each of all the clones. A purity test by CBB staining was performed, and as a result, no band other than that of the protein of interest was detected at all. Thus, it was recognized that purified antibodies were prepared at high purities.

### (3) Reactivity of Monoclonal Antibodies

The 10 clones selected in (2) above were each investigated for its reactivity against S100A8/A9 heterodimer, S100A8, or S100A9 and subclass, which are shown in Table 1.

**Table 1**

| Clone No. | Reactivity | | | Subclass |
|---|---|---|---|---|
| | S 1 00A8/A9 | S 1 00A8 | S100A9 | |
| 26 | ∘ | × | ∘ | IgG1 κ |
| 42 | ∘ | × | × | IgG2b κ |
| 45 | ∘ | × | × | IgG1 κ |
| 85 | ∘ | × | × | IgG2b κ |
| 108 | ∘ | × | ∘ | IgG1 κ |
| 213 | ∘ | ∘ | × | IgG2b κ |
| 219 | ∘ | × | × | IgG2b κ |
| 235 | ∘ | × | ∘ | IgG2b κ |
| 258 | ∘ | ∘ | × | IgG2b κ |
| 260 | ∘ | × | ∘ | IgG1 κ |

### (Example 2) Screening for Neutralizing Antibodies

In this Example, for the monoclonal antibodies produced from the 160 clones of hybridomas generated and selected in Example 1, their influences on the production amounts of S100A8/A9-induced inflammatory cytokines were investigated. Through use of human keratinocytes in which inflammatory cytokines were strongly induced by S100A8/A9, the S100A8/A9 signal-suppressing effect of each antibody was evaluated with the mRNA expression amounts of the inflammatory cytokines serving as indicators. Specifically, 30 ng/mL of purified S100A8/A9 and each anti-S100A8/A9 monoclonal antibody purified with the Protein G column from 1 mL of the culture supernatant of each of the 160 clones of hybridomas were added to keratinocytes (normal human keratinocytes: NHKs), and after culture at 37°C for 3 hours, the cells were recovered, followed by real-time quantitative PCR (qPCR) analysis for the respective mRNA expression amounts of TNF-α, IL-6, and IL-8.

The real-time quantitative PCR (qPCR) analysis was performed using a LightCycler rapid thermal cycler system (ABI 7900HT; Applied Biosystems). Measurement was performed using forward (Fwd) and reverse (Rev) primers having the following base sequences.

| | |
|---|---|
| For TNFα measurement | Fwd: GACAAGCCTGTAGCCCATGT (SEQ ID NO: 1) |
| | Rev: TCTCAGCTCCACGCCATT (SEQ ID NO: 2) |
| For IL-6 measurement | Fwd: CTTCCCTGCCCCAGTACC (SEQ ID NO: 3) |
| | Rev: CTGAAGAGGTGAGTGGCTGTC (SEQ ID NO: 4) |
| For IL-8 measurement | Fwd: AGACAGCAGAGCACACAAGC (SEQ ID NO: 5) |
| | Rev: AGGAAGGCTGCCAAGAGAG (SEQ ID NO: 6) |

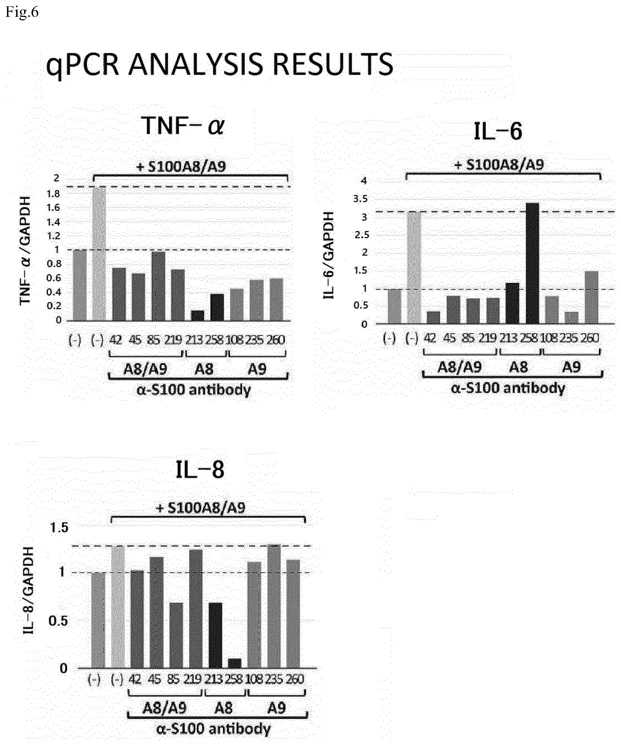

As the results of the foregoing, measurement results of the S100A8/A9 (abbreviated simply as "A8/A9")-induced inflammatory cytokines (TNF-α, IL-6, and IL-8) in the presence of the 10 selected clones (Clone Nos.: 26, 42, 45, 85, 108, 213, 219, 235, 258, and 260) are shown in FIG. 6. On the basis of the results, five kinds of antibodies having particularly high suppressive capacities (one kind of antibody reactive to S100A8 (abbreviated simply as "A8"), two kinds of antibodies reactive to S100A9 (abbreviated simply as "A9"), and two kinds of antibodies reactive only to an S100A8/A9 complex (abbreviated simply as "A8/A9")) were selected. In addition, "α-S100A8/A9 antibody" in FIG. 6 means anti-S100A8/A9 monoclonal antibody.

### (Example 3) Amino Acid Sequences of Variable Regions of Selected Antibodies

For the five kinds of anti-S100A8/A9 monoclonal antibodies (Clone Nos.: 45, 85, 235, 258, and 260) selected by the screening described above, the sequences of the variable regions of their heavy chains and light chains were analyzed.

### VH-CDR

Clone No. 45: CDR H1: SYWMQ (SEQ ID NO: 7)
Clone No. 45: CDR H2: AIYPGDGDTRDTQKFKG (SEQ ID NO: 8)
Clone No. 45: CDR H3: MAGYNYDNDY (SEQ ID NO: 9)
Clone No. 85: CDR H1: SGYNWH (SEQ ID NO: 10)
Clone No. 85: CDR H2: YIQYSGSTNYNPSLKS (SEQ ID NO: 11)
Clone No. 85: CDR H3: ALRYDYSWFAY (SEQ ID NO: 12)
Clone No. 235: CDR H1: NFWMN (SEQ ID NO: 13)
Clone No. 235: CDR H2: QIYPGKSDTNYNGKFKG (SEQ ID NO: 14)
Clone No. 235: CDR H3: WGAYYKYGGSYFDY (SEQ ID NO: 15)
Clone No. 258: CDR H1: TASMGVS (SEQ ID NO: 16)
Clone No. 258: CDR H2: HIYWDDDKRYNPSLKS (SEQ ID NO: 17)
Clone No. 258: CDR H3: RPLGYFDV (SEQ ID NO: 18)
Clone No. 260: CDR H1: NYGVH (SEQ ID NO: 19)
Clone No. 260: CDR H2: VVWAGGSTNYNSALMS (SEQ ID NO: 20)
Clone No. 260: CDR H3: ARDYYGYDGYFGA (SEQ ID NO: 21)

### VL-CDR

Clone No. 45: CDR L1: KASQDINKYIA (SEQ ID NO: 22)
Clone No. 45: CDR L2: YTSTLQP (SEQ ID NO: 23)
Clone No. 45: CDR L3: LQYDNLRT (SEQ ID NO: 24)
Clone No. 85: CDR L1: KASQDVSTAVA (SEQ ID NO: 25)
Clone No. 85: CDR L2: SASYRYT (SEQ ID NO: 26)
Clone No. 85: CDR L3: QQHYSTPLT (SEQ ID NO: 27)
Clone No. 235: CDR L1: SASQGISNYLN (SEQ ID NO: 28)
Clone No. 235: CDR L2: YTSSLHS (SEQ ID NO: 29)
Clone No. 235: CDR L3: QQYSKFPYT (SEQ ID NO: 30)
Clone No. 258: CDR L1: KASQDINNYIS (SEQ ID NO: 31)
Clone No. 258: CDR L2: YTSTLQP (SEQ ID NO: 32)
Clone No. 258: CDR L3: LQYDNLLWT (SEQ ID NO: 33)
Clone No. 260: CDR L1: KASQDINSYLT (SEQ ID NO: 34)
Clone No. 260: CDR L2: RANRLVD (SEQ ID NO: 35)
Clone No. 260: CDR L3: LQYDEFPLT (SEQ ID NO: 36)

### (Example 4) Generation of Anti-S100A8/A9 Chimeric Antibody

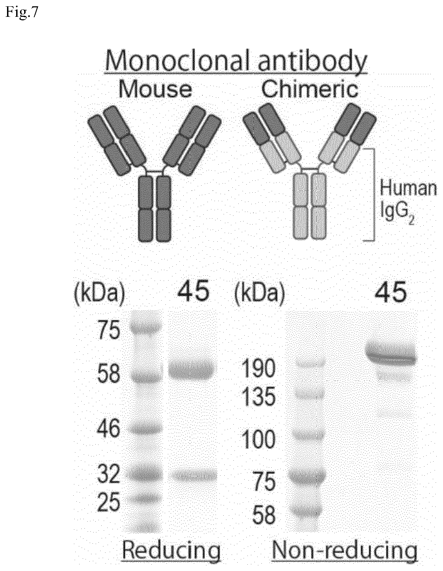

In this Example, a chimeric antibody having the Fc portion of human IgG₂ fused to the Fab domain of the anti-S100A8/A9 monoclonal antibody (Clone No. 45) was generated. Sequence analysis and CDR analysis of the variable regions of the heavy chain and light chain of the anti-S100A8/A9 monoclonal antibody (Clone No. 45) were performed, and a stable expression vector for CHO cells having incorporated therein sequences recombined with variable regions of human IgG₂ was generated and transduced into CHO cells in combination with a gene for the Fc portion of human IgG₂. Thus, the anti-S100A8/A9 chimeric antibody was stably generated (see FIG. 7). The antibody was generated by a method described in WO 2017/061354 A1.

### Example 5) Proliferation Enhancement of Fibroblasts by S100A8/A9

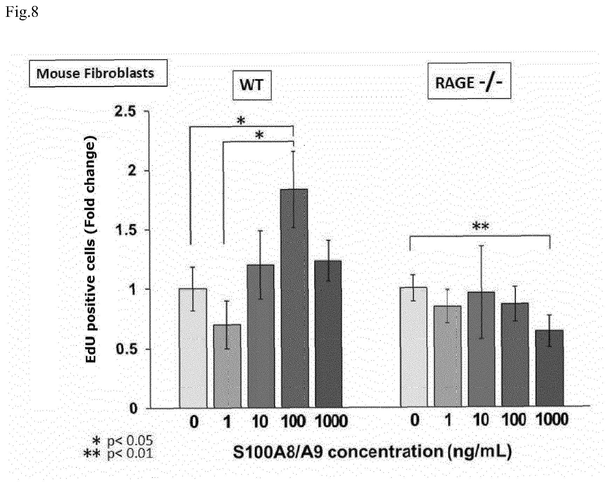

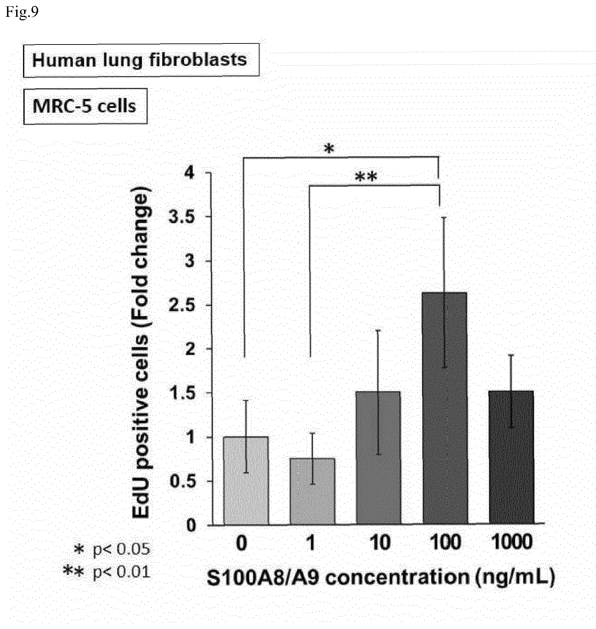

As a cause of pulmonary fibrosis, there is given abnormal proliferation of fibroblasts. In view of this, S100A8/A9-dependent proliferation of fibroblasts was investigated. Proliferation enhancement through S100A8/A9 addition of embryo fibroblasts (mouse embryo fibroblasts: MEFs) derived from a B6 mouse (wild type: WT) and a B6 mouse (RAGE-/-) in which RAGE, a major receptor for S100A8/A9 in the lungs, had been knocked out, was determined by a 5-ethynyl-2'-deoxyuridine (EdU) staining method (see FIG. 8). Proliferation enhancement through S100A8/A9 addition of MRC-5 cells, which were normal human embryonic lung tissue-derived fibroblasts (human lung fibroblasts), was similarly determined by the EdU staining method (see FIG. 9). Specifically, the determination was performed by the following method. First, fibroblasts that had been cultured in 10% fetal bovine serum (FBS)-containing GIBCO^{™} DMEM/F-12 (Dulbecco's Modified Eagle Medium/Nutrient Mixture F-12) medium were each seeded into a 6-well plate at 2×10⁵ cells/well. After 24 hours of culture, the medium was changed to a serum-free medium. After further culture for 24 hours, the medium was changed to a 0.5% FBS-containing medium, and S100A8/A9 was added at various concentrations. After further culture for 5 hours, EdU was added, and 1 hour later, the cells stained with EdU were recognized with a microscope. As shown in FIG. 8 and FIG. 9, through the addition of S100A8/A9, proliferation enhancement was recognized at an S100A8/A9 concentration of up to 100 ng/mL, and the proliferation rate was reduced at 1,000 ng/mL.

It was recognized from the above-mentioned results that S100A8/A9 showed a growth factor-like action on fibroblasts. Meanwhile, no proliferation enhancement was recognized in the RAGE knockout B6 mouse (RAGE-/-)-derived cells. Thus, it was suggested that S100A8/A9 promoted cell proliferation via RAGE, a receptor on fibroblasts.

### (Example 6) Evaluation of S100A8/A9-dependent NF-κB Signal Activation and NF-κB Signal Suppression by Anti-S 100A8/A9 Antibody in Fibroblasts

A transcription factor NF-κB present downstream of the S1 00A8/A9 receptor RAGE induces the expression of various inflammatory cytokines through its activation. In order to evaluate S100A8/A9-dependent activation of an NF-κB signal in fibroblasts, interaction between NF-κB and a biotin-labeled NF-κB-binding DNA probe was detected using gel shift assay (electrophoretic mobility shift assay: EMSA). Next, the action of the anti-S100A8/A9 antibody on NF-κB induced by S100A8/A9 was similarly determined by gel shift assay.

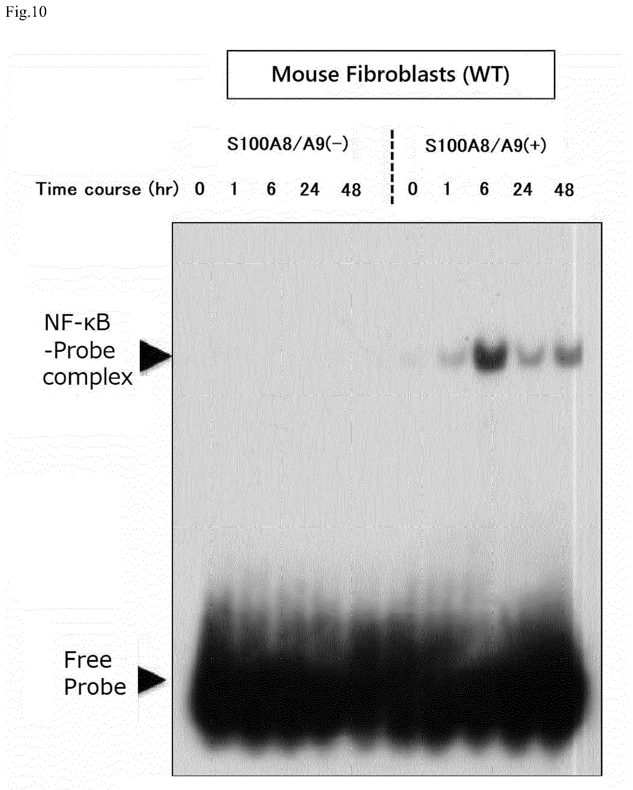

Specifically, the determination was performed by the following method. 5×10⁵ B6 mouse (WT)-derived fibroblasts that had been cultured in 10% FBS-containing GIBCO^{™}DMEM/F-12 medium were seeded into a 10 cm Petri dish, and 24 hours later, the medium was changed to a serum-free medium. After further culture for 6 hours, the medium was changed to a 0.5% FBS-containing medium, a control buffer (PBS) or 100 ng/mL S100A8/A9 was added, and 0 hours, 1 hour, 6 hours, 24 hours, or 48 hours later, the cells were washed with PBS and then recovered. The cells were suspended in 100 µL of M-PER (Mammalian Protein Extraction Reagent) from Thermo Scientific to prepare a cell lysate. A biotin-labeled NF-κB-binding DNA probe (NF-κB consensus binding motif 5'-agttgaGGGGACTTTCCcaggc-3' (SEQ ID NO: 37)) and the cell lysate were mixed, and the mixture was subjected to a reaction on ice for 30 minutes, followed by native gel electrophoresis (Native-PAGE). The gel was transferred to a nitrocellulose membrane, crosslinked by UV irradiation, and then blocked, followed by a reaction with Avidin-HRP and chemiluminescence detection. As a result, it was recognized that the activity of NF-κB was induced in an S100A8/A9-dependent manner, and the activity peaked 6 hours after the addition (see FIG. 10).

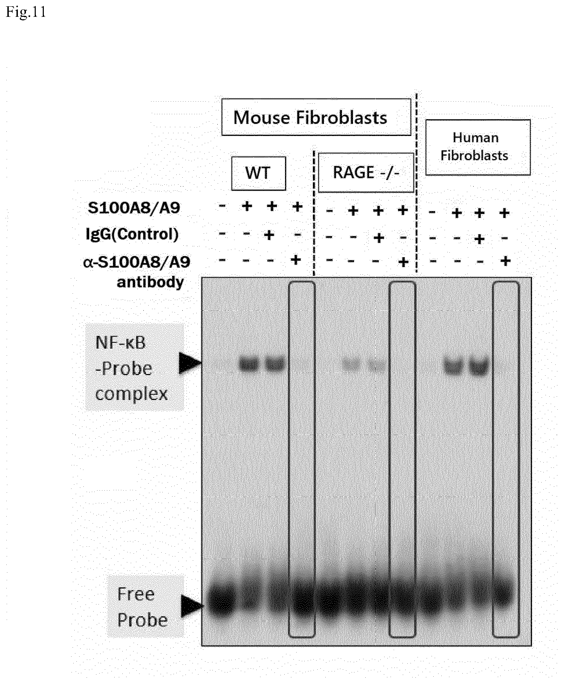

Next, gel shift assay was performed using the B6 mouse (WT)- and RAGE knockout B6 mouse (RAGE-/-)-derived embryo fibroblasts (MEFs) and the normal human embryonic lung tissue-derived fibroblasts (MRC-5) to determine the action of the anti-S 1 00A8/A9 antibody on NF-κB induced by S100A8/A9. 5×10⁵ each of mouse-derived fibroblasts and 1.5×10⁵ MRC-5 cells that had been cultured in 10% FBS-containing GIBCO^{™}DMEM/F-12 medium were each seeded into a 10 cm Petri dish, and 24 hours later, the medium was changed to a serum-free medium. After 6 hours of culture, the medium was changed to a 0.5% FBS-containing medium, a control buffer, 100 ng/mL S100A8/A9, 1 µg/mL IgG (control), or 1 µg/mL anti-S100A8/A9 antibody (α-S100A8/A9 antibody) was added in combinations shown in FIG. 11, and after further culture for 6 hours, cell lysates were recovered. Then, an experiment was performed by the same technique as described above. As a result, in the B6 mouse (WT) embryo fibroblasts (MEFs) and the MRC-5 cells, which were normal human embryonic lung tissue-derived fibroblasts, the activity of NF-κB was found in the systems containing no anti-S 100A8/A9 antibody, but the suppression of the activity of NF-κB was recognized in the systems containing the anti-S 1 00A8/A9 antibody (see FIG. 11). It was recognized that, in the embryo fibroblasts (MEFs) derived from the RAGE knockout B6 mouse (RAGE-/-), the NF-κB signal was slightly activated by stimulation with a 100A8/A9 complex. Incidentally, it was conceived that the slight activation of the NF-κB signal was due to receptors other than RAGE (EMMPRIN, NPTNβ, MCAM, and ALCAM identified as S100A8/A9 receptors by the inventors of the present invention). The activation of the NF-κB signal was completely suppressed by the anti-S 100A8/A9 antibody.

The anti-S 100A8/A9 antibody is specified as α-S100A8/A9 antibody in FIG. 11. α-S100A8/A9 antibody means the anti-S100A8/A9 monoclonal antibody (Clone No. 45), and IgG (control) means a mouse IgG isotype control. The same applies to α-S100A8/A9 antibody and IgG (control) in each of the figures mentioned in the following Examples.

### (Example 7) Evaluation of Expression Induction of α-SMA and Collagen by S100A8/A9 and S100A8/A9-induced α-SMA and Collagen Expression-suppressing Abilities of Anti-S 1 00A8/A9 Antibody in Fibroblasts

In the pathological condition of pulmonary fibrosis, the differentiation of activated fibroblasts into myofibroblasts induces and is accompanied by excessive deposition of collagen and extracellular matrix components to exacerbate pulmonary fibrosis. Nintedanib, which is an approved therapeutic drug for idiopathic pulmonary fibrosis (IPF), inhibits the proliferation of fibroblasts and their differentiation into myofibroblasts. In view of this, S100A8/A9 was determined to be a risk factor inducing differentiation from fibroblasts to myofibroblasts on the basis of the expression of α-smooth muscle actin (a-SMA) as a myofibroblast marker, and the action of the anti-S 100A8/A9 antibody on α-SMA expression was determined.

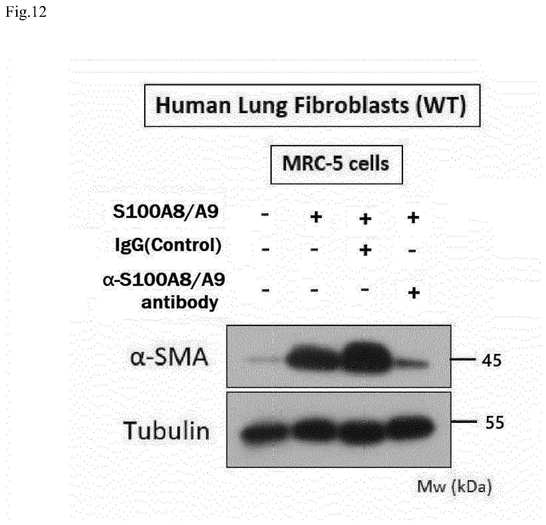

Specifically, the determination was performed by the following method. 5×10⁴ normal human embryonic lung tissue-derived fibroblasts (MRC-5), and 3×10⁵ each of B6 mouse (WT)- and RAGE knockout B6 mouse (RAGE-/-)-derived embryo fibroblasts that had been cultured in 10% FBS-containing GIBCO^{™}DMEM/F-12 medium were each seeded into a 10 cm Petri dish, and 24 hours later, the medium was changed to a serum-free medium. After further culture for 6 hours, the medium was changed to a 0.5% FBS-containing medium, a control buffer, 100 ng/mL S100A8/A9, 1 µg/mL IgG (control), or 1 µg/mL anti-S100A8/A9 antibody was added in combinations shown in FIG. 12 and FIG. 13, and 48 hours later, cell lysates were recovered.

The recovered cell lysates were subjected to western blotting to detect proteins. For the normal human embryonic lung tissue-derived fibroblasts (MRC-5), proteins were detected using an anti-α-SMA monoclonal antibody and an anti-tubulin antibody serving as a control. In the MRC-5 cells, S100A8/A9 strongly induced the expression of α-SMA, and the induced expression increase was remarkably suppressed by co-culture with the anti-S100A8/A9 antibody (see FIG. 12). For the B6 mouse (WT)- and RAGE knockout B6 mouse (RAGE-/-)-derived embryo fibroblasts, proteins were detected using the anti-aSMA monoclonal antibody, a biotin-labeled probe capable of specifically binding to a collagen chain denatured by a collagenase, mechanical damage to a connective tissue, or the like, and the anti-tubulin antibody serving as a control.

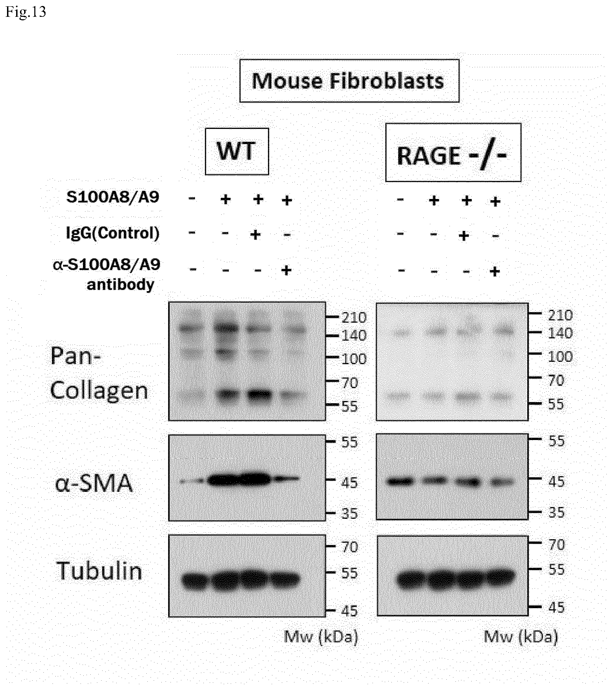

In the B6 mouse (WT)-derived embryo fibroblasts, S100A8/A9 strongly induced the expression of α-SMA and the expression of collagen. The increases in expression of α-SMA and collagen induced by S100A8/A9 were remarkably suppressed by co-culture with the anti-S100A8/A9 antibody (see FIG. 13). Meanwhile, for the RAGE knockout B6 mouse (RAGE-/-)-derived embryo fibroblasts, increases in expression amounts of α-SMA and collagen by S100A8/A9 were not recognized. Thus, it was suggested that S100A8/A9 induced the expression of α-SMA and collagen via RAGE, a receptor on fibroblasts.

Thus, S100A8/A9 was determined to be a risk factor inducing differentiation from fibroblasts to myofibroblasts. Besides, suppressing actions of the anti-S 1 00A8/A9 antibody were recognized on the expression of the myofibroblast markers α-SMA and collagen (see FIG. 12 and FIG. 13). Those results suggested a prophylactic effect of the anti-S100A8/A9 antibody on pulmonary fibrosis.

### (Example 8) Lung Injury-suppressing Effect of Anti-S100A8/A9 Antibody in Pulmonary Fibrosis Model Intratracheally Injected with Bleomycin

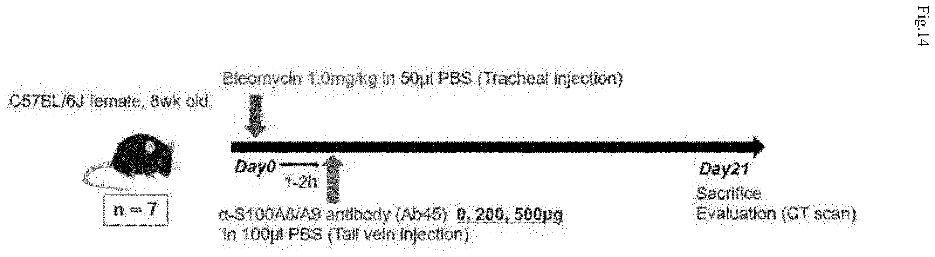

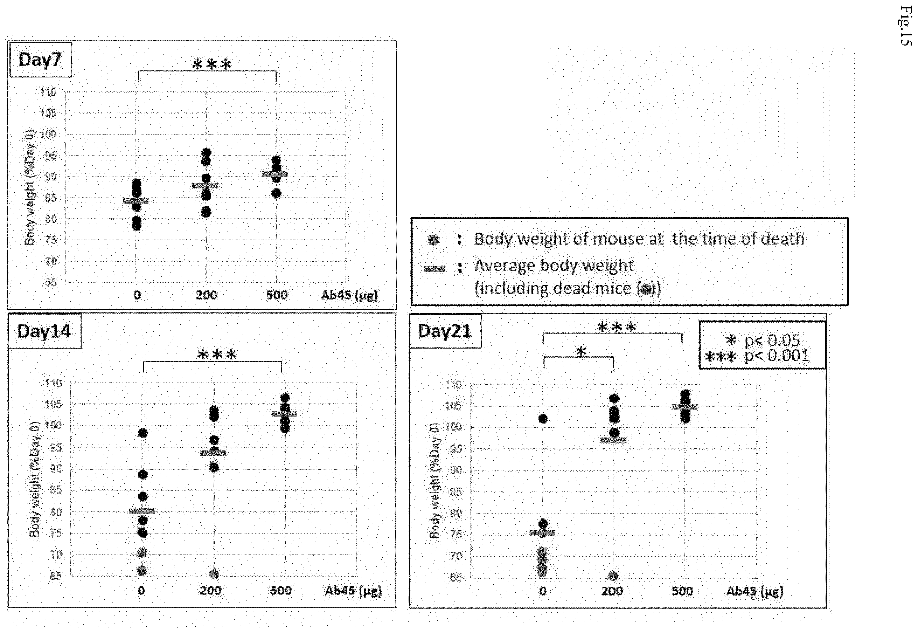

The lung injury-suppressing effect of the anti-S100A8/A9 antibody in a bleomycin-induced pulmonary fibrosis model was investigated. In accordance with a protocol illustrated in FIG. 14, seven female C57BL/6J (8-week-old) mice per group were intratracheally injected with 50 µl of PBS containing bleomycin at 1.0 mg/kg body weight of the mice to generate lung injury model mice. After from 1 hour to 2 hours from the bleomycin injection, a PBS buffer (control group: 0 µg), or 200 µg and 500 µg of the anti-S100A8/A9 antibody was injected into the tail vein. After 7 days, 14 days, and 21 days from the bleomycin injection, changes in body weight of the lung injury model mice were observed, and as a result, body weight reduction-suppressing effects on the mice were significantly found in the group injected with 500 µg of the anti-S 100A8/A9 antibody as compared to the control group on day 7 and day 14. On day 21, a body weight reduction-suppressing effect on the mice was significantly found in each of the group injected with 200 µg of the anti-S100A8/A9 antibody and the group injected with 500 µg thereof as compared to the control group (FIG. 15).

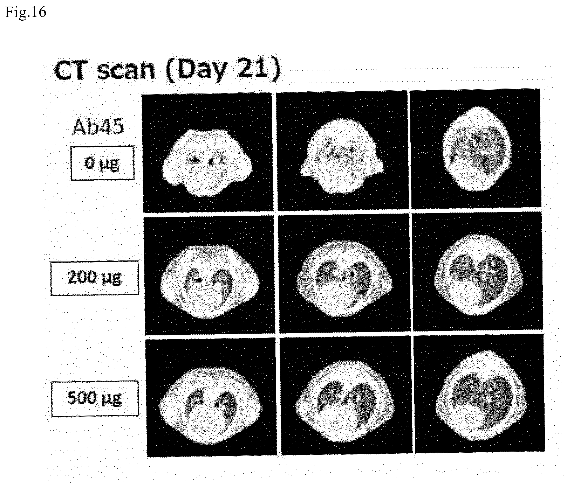

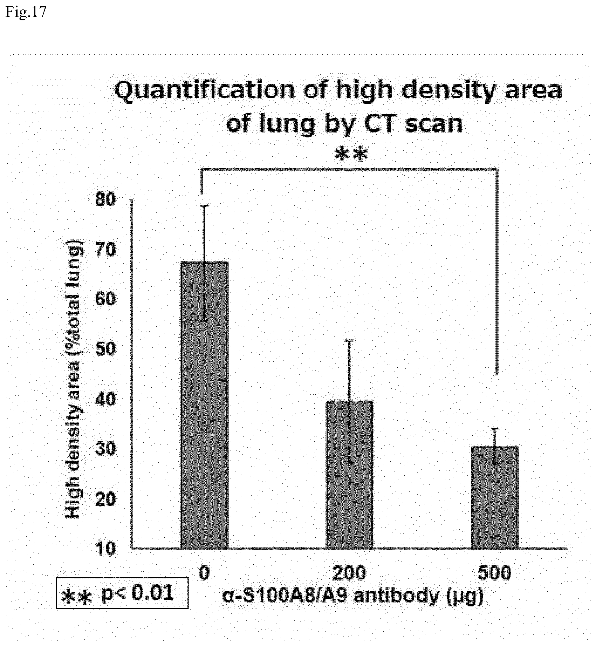

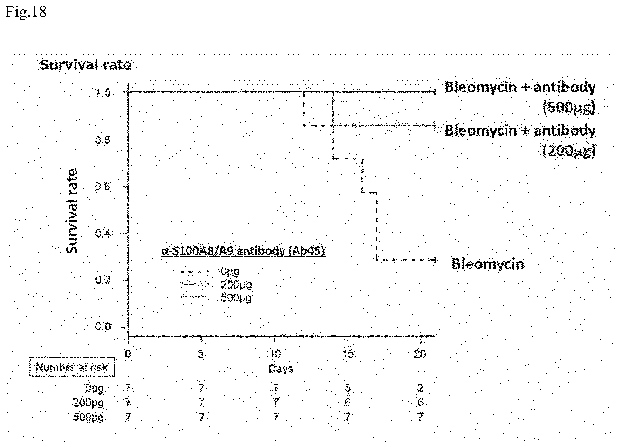

Further, on day 21 after the bleomycin injection, the lung injury-suppressing effect of the anti-S100A8/A9 antibody was investigated by CT scanning. Typical CT scan images are shown in FIG. 16. In addition, a graph quantifying the high density areas of the CT scan images (regions recognized as white in the lung tissues of the CT scan images) through imaging is shown in FIG. 17. In the group injected with 500 µg of the anti-S100A8/A9 antibody, a significant suppressing effect was observed on the injury/fibrosis of the lung tissue on day 21 after the bleomycin injection. Further, the survival rates of the mice in this experiment are shown in FIG. 18. In the group injected with 500 µg of the anti-S100A8/A9 antibody, no dead mouse was found, but in the group injected with no antibody, five out of the seven mice were found to be dead. Numbers under "Number at risk" shown in the lower part of FIG. 18 represent the numbers of surviving mice.

Thus, the anti-S 100A8/A9 antibody showed an excellent therapeutic effect on pulmonary fibrosis also in the *in vivo* system using the intratracheally injected pulmonary fibrosis model.

### (Example 9) Suppressing Effect of Anti-S100A8/A9 Antibody on TMPRSS2 Expression in Human Lung Cells

As described in Example 8, it was found that the anti-S100A8/A9 antibody showed an excellent therapeutic effect on pulmonary fibrosis. The inventors of the present invention continued investigating further pharmacological actions of the anti-S 1 00A8/A9 antibody, and in doing so, examined the influence of the anti-S100A8/A9 antibody on TMPRSS2 expression in view of the fact that TMPRSS2, which is a host protease expressed in the respiratory epithelium, is an important protein in infection with SARS-CoV-2. That is, with use of human lung cells, S100A8/A9 protein-dependent changes in TMPRSS2 expression and the influence of the anti-S100A8/A9 antibody on TMPRSS2 expression were examined using the anti-S100A8/A9 monoclonal antibody (Clone No. 45) as the anti-S100A8/A9 antibody.

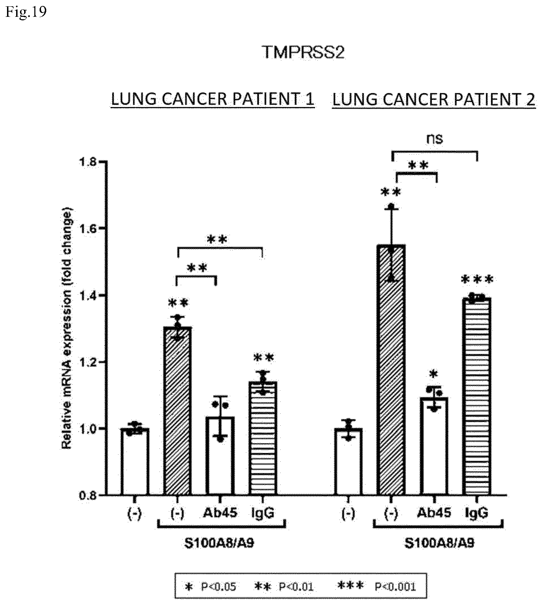

That is, a normal part of a lung tissue excised during human lung cancer surgery was cut into a piece having a diameter of about 3 mm, which was treated with a collagenase in a serum-free medium at 4°C for 24 hours to disperse lung tissue-derived cells. The thus obtained cells were suspended in a serum-free medium, and then 1 µg/mL purified S100A8/A9 and 10 µg/mL anti-S100A8/A9 antibody (Clone No. 45) or 10 µg/mL control IgG were added. After culture at 37°C for 6 hours, the cells were recovered, and RNA was extracted and subjected to real-time quantitative PCR (qPCR) analysis for TMPRESS2 (FIG. 19). For the real-time quantitative PCR (qPCR) analysis, measurement was performed using StepOnePlus Realtime PCR (Applied Biosystems), and using forward (Fwd) and reverse (Rev) primers having the following base sequences.
Primer sequences for TMPRSS2 measurement
Fwd: GATGACAGCGGATCCACCAG (SEQ ID NO: 38)
Rev: CCGCAGGCTATACAGCGTAA (SEQ ID NO: 39)

As a result, it was revealed that TMPRSS2 activated SARS-CoV-2 bound to an ACE2 receptor present on the surface of cells to enhance the efficiency of penetration into the cells (cleavage activation of the spike protein on the envelope of the virus). Then, the inventors of the present invention focused their attention on the expression of TMPRSS2 in human lung cells, and analyzed the mRNA expression thereof. As a result, it was revealed that S100A8/A9 significantly induced the expression of TMPRSS2 in the human lung cells, and the anti-S100A8/A9 antibody remarkably suppressed the induced expression.

On the basis of the above-mentioned findings, it was suggested that the anti-S100A8/A9 antibody was also useful for protection against SARS-CoV-2 infection through suppression of TMPRSS2 expression in addition to having a suppressing effect on a cytokine storm induced by the SARS-CoV-2 virus in the treatment of COVID-19.

### Industrial Applicability

As described above, the prophylactic and/or therapeutic agent for an inflammatory pulmonary disease of the present invention can effectively prevent and/or treat an inflammatory pulmonary disease by blocking the interaction of S100A8/A9, whose expression is caused to increase by the inflammatory pulmonary disease, with RAGE, which is a receptor therefor and highly expressed in alveolar epithelial cells serving as the origin of a pathological condition, and a plurality of S100A8/A9 receptors expressed in lung fibroblasts, to thereby suppress inflammatory cytokine release from alveolar epithelial cells and the proliferation of lung fibroblasts, and suppress the differentiation of activated fibroblasts into myofibroblasts. In addition, the prophylactic and/or therapeutic agent for an inflammatory pulmonary disease of the present invention may also be suitably used as a prophylactic and/or therapeutic agent for COVID-19. The industrial usefulness of the present invention, which achieves such remarkable actions and effects, is immeasurable.

## Claims

1. A prophylactic and/or therapeutic agent for an inflammatory pulmonary disease, comprising an antibody or an antibody fragment as an active ingredient, the antibody or the antibody fragment having antigen-binding activity for a heterodimer of an S100A8 protein and an S 1 00A9 protein.

2. The prophylactic and/or therapeutic agent for an inflammatory pulmonary disease according to claim 1, wherein the antibody or the antibody fragment has antigen-binding activity for the heterodimer higher than antigen-binding activity for an S100A8 monomer.

3. The prophylactic and/or therapeutic agent for an inflammatory pulmonary disease according to claim 1 or 2, wherein the antibody or the antibody fragment has antigen-binding activity for the heterodimer of S100A8 and S 100A9, and is free of antigen-binding activity for an S100A8 monomer and/or an S100A9 monomer.

4. The prophylactic and/or therapeutic agent for an inflammatory pulmonary disease according to any one of claims 1 to 3, wherein the antigen-binding activity is a neutralizing affinity.

5. The prophylactic and/or therapeutic agent for an inflammatory pulmonary disease according to any one of claims 1 to 4, wherein the antibody or the antibody fragment is a monoclonal antibody or a monoclonal antibody fragment.

6. The prophylactic and/or therapeutic agent for an inflammatory pulmonary disease according to claim 5, wherein a subclass of the monoclonal antibody is selected from the group consisting of IgG₁, IgG₂, IgG₃, and IgG₄.

7. The prophylactic and/or therapeutic agent for an inflammatory pulmonary disease according to any one of claims 1 to 6,
wherein the antibody or the antibody fragment as the active ingredient is an antibody or an antibody fragment comprising:
heavy chain variable regions comprising a heavy chain variable region 1 (CDR HI), a heavy chain variable region 2 (CDR H2), and a heavy chain variable region 3 (CDR H3); and light chain variable regions comprising a light chain variable region 1 (CDR L1), a light chain variable region 2 (CDR L2), and a light chain variable region 3 (CDR L3),
wherein the heavy chain variable region 1 (CDR H1) comprises any one of the amino acid sequence set forth in SEQ ID NO: 7, SEQ ID NO: 10, SEQ ID NO: 13, SEQ ID NO: 16, or SEQ ID NO: 19, or the amino acid sequence having one or a plurality of amino acids deleted, added, substituted, or inserted in SEQ ID NO: 7, 10, 13, 16, or 19,
wherein the heavy chain variable region 2 (CDR H2) comprises any one of the amino acid sequence set forth in SEQ ID NO: 8, SEQ ID NO: 11, SEQ ID NO: 14, SEQ ID NO: 17, or SEQ ID NO: 20, or the amino acid sequence having one or a plurality of amino acids deleted, added, substituted, or inserted in SEQ ID NO: 8, 11, 14, 17, or 20,
wherein the heavy chain variable region 3 (CDR H3) comprises any one of the amino acid sequence set forth in SEQ ID NO: 9, SEQ ID NO: 12, SEQ ID NO: 15, SEQ ID NO: 18, or SEQ ID NO: 21, or the amino acid sequence having one or a plurality of amino acids deleted, added, substituted, or inserted in SEQ ID NO: 9, 12, 15, 18, or 21,
wherein the light chain variable region 1 (CDR L1) comprises any one of the amino acid sequence set forth in SEQ ID NO: 22, SEQ ID NO: 25, SEQ ID NO: 28, SEQ ID NO: 31, or SEQ ID NO: 34, or the amino acid sequence having one or a plurality of amino acids deleted, added, substituted, or inserted in SEQ ID NO: 22, 25, 28, 31, or 34,
wherein the light chain variable region 2 (CDR L2) comprises any one of the amino acid sequence set forth in SEQ ID NO: 23, SEQ ID NO: 26, SEQ ID NO: 29, SEQ ID NO: 32, or SEQ ID NO: 35, or the amino acid sequence having one or a plurality of amino acids deleted, added, substituted, or inserted in SEQ ID NO: 23, 26, 29, 32, or 35, and
wherein the light chain variable region 3 (CDR L3) comprises any one of the amino acid sequence set forth in SEQ ID NO: 24, SEQ ID NO: 27, SEQ ID NO: 30, SEQ ID NO: 33, or SEQ ID NO: 36, or the amino acid sequence having one or a plurality of amino acids deleted, added, substituted, or inserted in SEQ ID NO: 24, 27, 30, 33, or 36.

8. The prophylactic and/or therapeutic agent for an inflammatory pulmonary disease according to claim 7,
wherein the heavy chain variable region 1 (CDR H1) comprises any one of the amino acid sequence set forth in SEQ ID NO: 7, or the amino acid sequence having one or a plurality of amino acids deleted, added, substituted, or inserted in the amino acid sequence set forth in SEQ ID NO: 7,
wherein the heavy chain variable region 2 (CDR H2) comprises any one of the amino acid sequence set forth in SEQ ID NO: 8, or the amino acid sequence having one or a plurality of amino acids deleted, added, substituted, or inserted in the amino acid sequence set forth in SEQ ID NO: 8,
wherein the heavy chain variable region 3 (CDR H3) comprises any one of the amino acid sequence set forth in SEQ ID NO: 9, or the amino acid sequence having one or a plurality of amino acids deleted, added, substituted, or inserted in the amino acid sequence set forth in SEQ ID NO: 9,
wherein the light chain variable region 1 (CDR L1) comprises any one of the amino acid sequence set forth in SEQ ID NO: 22, or the amino acid sequence having one or a plurality of amino acids deleted, added, substituted, or inserted in the amino acid sequence set forth in SEQ ID NO: 22,
wherein the light chain variable region 2 (CDR L2) comprises any one of the amino acid sequence set forth in SEQ ID NO: 23, or the amino acid sequence having one or a plurality of amino acids deleted, added, substituted, or inserted in the amino acid sequence set forth in SEQ ID NO: 23, and
wherein the light chain variable region 3 (CDR L3) comprises any one of the amino acid sequence set forth in SEQ ID NO: 24, or the amino acid sequence having one or a plurality of amino acids deleted, added, substituted, or inserted in the amino acid sequence set forth in SEQ ID NO: 24.

9. The prophylactic and/or therapeutic agent for an inflammatory pulmonary disease according to claim 7,
wherein the heavy chain variable region 1 (CDR H1) comprises any one of the amino acid sequence set forth in SEQ ID NO: 10, or the amino acid sequence having one or a plurality of amino acids deleted, added, substituted, or inserted in the amino acid sequence set forth in SEQ ID NO: 10,
wherein the heavy chain variable region 2 (CDR H2) comprises any one of the amino acid sequence set forth in SEQ ID NO: 11, or the amino acid sequence having one or a plurality of amino acids deleted, added, substituted, or inserted in the amino acid sequence set forth in SEQ ID NO: 11,
wherein the heavy chain variable region 3 (CDR H3) comprises any one of the amino acid sequence set forth in SEQ ID NO: 12, or the amino acid sequence having one or a plurality of amino acids deleted, added, substituted, or inserted in the amino acid sequence set forth in SEQ ID NO: 12,
wherein the light chain variable region 1 (CDR L1) comprises any one of the amino acid sequence set forth in SEQ ID NO: 25, or the amino acid sequence having one or a plurality of amino acids deleted, added, substituted, or inserted in the amino acid sequence set forth in SEQ ID NO: 25,
wherein the light chain variable region 2 (CDR L2) comprises any one of the amino acid sequence set forth in SEQ ID NO: 26, or the amino acid sequence having one or a plurality of amino acids deleted, added, substituted, or inserted in the amino acid sequence set forth in SEQ ID NO: 26, and
wherein the light chain variable region 3 (CDR L3) comprises any one of the amino acid sequence set forth in SEQ ID NO: 27, or the amino acid sequence having one or a plurality of amino acids deleted, added, substituted, or inserted in the amino acid sequence set forth in SEQ ID NO: 27.

10. The prophylactic and/or therapeutic agent for an inflammatory pulmonary disease according to claim 7,
wherein the heavy chain variable region 1 (CDR H1) comprises any one of the amino acid sequence set forth in SEQ ID NO: 13, or the amino acid sequence having one or a plurality of amino acids deleted, added, substituted, or inserted in the amino acid sequence set forth in SEQ ID NO: 13,
wherein the heavy chain variable region 2 (CDR H2) comprises any one of the amino acid sequence set forth in SEQ ID NO: 14, or the amino acid sequence having one or a plurality of amino acids deleted, added, substituted, or inserted in the amino acid sequence set forth in SEQ ID NO: 14,
wherein the heavy chain variable region 3 (CDR H3) comprises any one of the amino acid sequence set forth in SEQ ID NO: 15, or the amino acid sequence having one or a plurality of amino acids deleted, added, substituted, or inserted in the amino acid sequence set forth in SEQ ID NO: 15,
wherein the light chain variable region 1 (CDR L1) comprises any one of the amino acid sequence set forth in SEQ ID NO: 28, or the amino acid sequence having one or a plurality of amino acids deleted, added, substituted, or inserted in the amino acid sequence set forth in SEQ ID NO: 28,
wherein the light chain variable region 2 (CDR L2) comprises any one of the amino acid sequence set forth in SEQ ID NO: 29, or the amino acid sequence having one or a plurality of amino acids deleted, added, substituted, or inserted in the amino acid sequence set forth in SEQ ID NO: 29, and
wherein the light chain variable region 3 (CDR L3) comprises any one of the amino acid sequence set forth in SEQ ID NO: 30, or the amino acid sequence having one or a plurality of amino acids deleted, added, substituted, or inserted in the amino acid sequence set forth in SEQ ID NO: 30.

11. The prophylactic and/or therapeutic agent for an inflammatory pulmonary disease according to claim 7,
wherein the heavy chain variable region 1 (CDR H1) comprises any one of the amino acid sequence set forth in SEQ ID NO: 16, or the amino acid sequence having one or a plurality of amino acids deleted, added, substituted, or inserted in the amino acid sequence set forth in SEQ ID NO: 16,
wherein the heavy chain variable region 2 (CDR H2) comprises any one of the amino acid sequence set forth in SEQ ID NO: 17, or the amino acid sequence having one or a plurality of amino acids deleted, added, substituted, or inserted in the amino acid sequence set forth in SEQ ID NO: 17,
wherein the heavy chain variable region 3 (CDR H3) comprises any one of the amino acid sequence set forth in SEQ ID NO: 18, or the amino acid sequence having one or a plurality of amino acids deleted, added, substituted, or inserted in the amino acid sequence set forth in SEQ ID NO: 18,
wherein the light chain variable region 1 (CDR L1) comprises any one of the amino acid sequence set forth in SEQ ID NO: 31, or the amino acid sequence having one or a plurality of amino acids deleted, added, substituted, or inserted in the amino acid sequence set forth in SEQ ID NO: 31,
wherein the light chain variable region 2 (CDR L2) comprises any one of the amino acid sequence set forth in SEQ ID NO: 32, or the amino acid sequence having one or a plurality of amino acids deleted, added, substituted, or inserted in the amino acid sequence set forth in SEQ ID NO: 32, and
wherein the light chain variable region 3 (CDR L3) comprises any one of the amino acid sequence set forth in SEQ ID NO: 33, or the amino acid sequence having one or a plurality of amino acids deleted, added, substituted, or inserted in the amino acid sequence set forth in SEQ ID NO: 33.

12. The prophylactic and/or therapeutic agent for an inflammatory pulmonary disease according to claim 7,
wherein the heavy chain variable region 1 (CDR H1) comprises any one of the amino acid sequence set forth in SEQ ID NO: 19, or the amino acid sequence having one or a plurality of amino acids deleted, added, substituted, or inserted in the amino acid sequence set forth in SEQ ID NO: 19,
wherein the heavy chain variable region 2 (CDR H2) comprises any one of the amino acid sequence set forth in SEQ ID NO: 20, or the amino acid sequence having one or a plurality of amino acids deleted, added, substituted, or inserted in the amino acid sequence set forth in SEQ ID NO: 20,
wherein the heavy chain variable region 3 (CDR H3) comprises any one of the amino acid sequence set forth in SEQ ID NO: 21, or the amino acid sequence having one or a plurality of amino acids deleted, added, substituted, or inserted in the amino acid sequence set forth in SEQ ID NO: 21,
wherein the light chain variable region 1 (CDR L1) comprises any one of the amino acid sequence set forth in SEQ ID NO: 34, or the amino acid sequence having one or a plurality of amino acids deleted, added, substituted, or inserted in the amino acid sequence set forth in SEQ ID NO: 34,
wherein the light chain variable region 2 (CDR L2) comprises any one of the amino acid sequence set forth in SEQ ID NO: 35, or the amino acid sequence having one or a plurality of amino acids deleted, added, substituted, or inserted in the amino acid sequence set forth in SEQ ID NO: 35, and
wherein the light chain variable region 3 (CDR L3) comprises any one of the amino acid sequence set forth in SEQ ID NO: 36, or the amino acid sequence having one or a plurality of amino acids deleted, added, substituted, or inserted in the amino acid sequence set forth in SEQ ID NO: 36.

13. The prophylactic and/or therapeutic agent for an inflammatory pulmonary disease according to any one of claims 1 to 12, wherein the prophylactic and/or therapeutic agent blocks interaction between S100A8 and/or S100A9 and RAGE, which is a receptor therefor, to suppress: expression of NF-κB, which is a transcription factor present downstream of RAGE and induces expression of inflammatory cytokines, and proliferation of lung fibroblasts; proliferation of activated fibroblasts; and differentiation of activated fibroblasts into myofibroblasts.

14. The prophylactic and/or therapeutic agent for an inflammatory pulmonary disease according to any one of claims 1 to 13, wherein the prophylactic and/or therapeutic agent serves as a prophylactic and/or therapeutic agent for COVID-19.
